(19) Europäisches Patentamt
European Patent Office
Office européen des brevets

(11) EP 4 755 868 A1

(12) **EUROPEAN PATENT APPLICATION**

(43) Date of publication:
**10.06.2026 Bulletin 2026/24**

(21) Application number: **25191305.9**

(22) Date of filing: **23.07.2025**

(51) International Patent Classification (IPC):
***C07C 43/225*** *(2006.01)* ***C08F 16/00*** *(2006.01)*

(52) Cooperative Patent Classification (CPC):
**C07C 43/225; C08F 16/00;** C07C 2603/18

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: **03.12.2024 KR 20240177586**

(71) Applicant: **SK Innovation Co., Ltd.**
**Seoul 03188 (KR)**

(72) Inventors:
- **KIM, Cheol Woo**
  **34124 Daejeon (KR)**
- **LEE, Dong Gun**
  **34124 Daejeon (KR)**
- **LEE, So Young**
  **34124 Daejeon (KR)**
- **CHO, Soo Min**
  **34124 Daejeon (KR)**
- **KIM, Jong Chan**
  **34124 Daejeon (KR)**
- **LEE, Jung A**
  **34124 Daejeon (KR)**

(74) Representative: **Prüfer & Partner mbB**
**Patentanwälte · Rechtsanwälte**
**Sohnckestraße 12**
**81479 München (DE)**

# (54) COMPOUND, ION CONDUCTIVE POLYMER MANUFACTURED BY THE SAME AND SEPARATOR INCLUDING THE SAME

(57) The present disclosure provides a compound including an organic group in which at least one hydrogen is substituted with a halogen atom, an ether group and a multi-aromatic ring, which is represented by Formula 1 below.

[Formula 1]

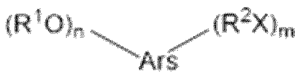

In Formula 1, m and n are each independently an integer of 1 to 6, $R^1$ is each independently an organic group having 1 to 10 carbon atoms, $R^2$ is each independently an organic group having 1 to 20 carbon atoms, which includes at least one of an alkylene group and/or an arylene group, X is each independently a halogen atom, and Ars is a multi-aromatic ring having 10 to 50 carbon atoms.

EP 4 755 868 A1

## Description

Field of the Invention

The present disclosure relates to a compound, an ion conductive polymer manufactured by the compound, a separator including the ion conductive polymer and a device including the separator.

Description of the Related Art

Generally, a separator is a barrier which exists between two different substances, materials or elements, and allows a specific substance to selectively pass therethrough. The separators may be classified into various types according to properties, materials, shapes, etc. thereof. In this case, an ion exchange membrane, one type in the separators, corresponds to a non-porous membrane, a symmetrical membrane, a single membrane, a homogeneous membrane, a hydrophilic membrane, a charged membrane, a liquid separator and other types of ion exchange membranes and separators.

The ion exchange membrane is a membrane having an ion exchange function for a specific ion, which is a specified and high-efficient type of separator. When introducing an ion exchange membrane into an electrolyte solution and applying a current thereto, functional groups having positive or negative charges on pore walls of the membrane attract only specific ions having opposite charges into the pores of the membrane, thereby retaining electrical neutrality. The specific ions that have been bound to the functional groups on the pores of the membrane pass through the membrane by continuously repeating binding to the functional groups on the pores and dissociating therefrom.

The ion exchange membranes may be classified into a cation exchange membrane (CEM), an anion exchange membrane (AEM), and a bipolar exchange membrane (BEM) according to characteristics thereof. The cation exchange membrane is a membrane which allows only cations to pass therethrough while anions serve as the fixed charges. The anion exchange membrane is a membrane which allows only anions to pass therethrough while cations serve as the fixed charges. The bipolar exchange membrane has a form in which cation and anion exchange membranes are coupled side-by-side, and allows both the cations and anions to selectively pass therethrough.

The ion exchange membrane may be used in various fields such as water treatment, chemistry, energy industries and the other technical fields. Since the ion exchange membrane should allow different specific charges to selectively pass therethrough, it is advantageous to have low electrical resistance and/or high ionic conductivity. In addition, the ion exchange membrane should preferably have excellent mechanical strength and/or be chemically stable in order to use continuously for a long period of time.

[Prior Art Document]

[Patent Document]

Korean Patent Laid-Open Publication No. 2018-0100079

[SUMMARY OF THE INVENTION]

An object of the present disclosure is to provide a compound for manufacturing an ion conductive polymer having improved electrochemical properties.

Another object of the present disclosure is to provide an ion conductive polymer and a separator manufactured from the compound.

In an aspect of the present disclosure, an ion conductive polymer is provided, wherein the ion conductive polymer includes a repeating unit which includes a polycyclic aromatic group and which bears electron withdrawing group(s). With a separator or ion exchange membrane with a conductive polymer having such structural configuration of repeating unit, ionic conductivity may be improved. The repeating unit may have a cation and an anion bound to the polycyclic aromatic group, and/or may have an alkoxy group bound to the polycyclic aromatic group. In further embodiments, the cation may be a quaternary ammonium, and the anion may be a halogen anion or a polyatomic anion, such as for example a chloride anion ($Cl^-$), a bromine anion ($Br^-$), an iodine anion ($I^-$), a hydroxide ion ($OH^-$), a sulfate anion ($SO_4^{2-}$), a carbonate anion ($CO_3^{2-}$), a bicarbonate anion ($HCO_3^-$), or a carboxylic acid anion ($RCO_2^-$). In further embodiments, the repeating unit may be defined by Formula 5 below or more specifically by any one of Formulas 6 to 8, or further specifically by any one of Formulas 6-1 to 8-1 below, respectively.

In another aspect of the present disclosure, an ion conductive polymer is provided, wherein the ion conductive polymer includes the aromatic group in the main chain in a repeating unit. With a separator or ion exchange membrane with a conductive polymer having such structural configuration of repeating unit, mechanical strength and chemical resistance

may be improved. The repeating unit may have a cation and an anion bound to the polycyclic aromatic group, and/or may have an alkoxy group bound to the polycyclic aromatic group. In further embodiments, the cation may be a quaternary ammonium, and the anion may be a halogen anion or a polyatomic anion, such as for example a chloride anion ($Cl^-$), a bromine anion ($Br^-$), an iodine anion ($I^-$), a hydroxide ion ($OH^-$), a sulfate anion ($SO_4^{2-}$), a carbonate anion ($CO_3^{2-}$), a bicarbonate anion ($HCO_3^-$), or a carboxylic acid anion ($RCO_2^-$). In further embodiments, the repeating unit may be defined by Formula 5 below or more specifically by any one of Formulas 6 to 8, or further specifically by any one of Formulas 6-1 to 8-1 below, respectively.

In another aspect of the present disclosure, an ion conductive polymer is provided, wherein the ion conductive polymer includes a quaternary ammonium bonded to the aromatic group via an organic group of aromatic and/or aliphatic chains in a repeating unit. In embodiments, at least two quaternary ammonium groups are bonded to a repeating unit (respectively the polycyclic aromatic group thereof), and/or the quaternary ammonium group(s) is/are respectively bonded to the repeating unit via hydrocarbon groups having a length of at least 2 carbon atoms, preferably 3 carbon atoms or more. With a separator or ion exchange membrane with a conductive polymer having such structural configuration of repeating unit, ion conductivity may be improved.

To achieve the above objects, according to an aspect of the present invention, there is provided a compound represented by Formula 1 below.

[Formula 1]

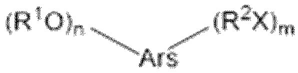

In Formula 1, m and n are each independently an integer of 1 to 6, $R^1$ is each independently an organic group having 1 to 10 carbon atoms, $R^2$ is each independently an organic group having 1 to 20 carbon atoms, which includes at least one of an alkylene group and/or an arylene group, X is each independently a halogen, and Ars is a polycyclic aromatic group having 10 to 50 carbon atoms. $R^1$ being each independently said organic group means that $R^1$ may be the same or different among the n groups. Further, $R^2$ being each independently said organic group means that $R^2$ may be the same or different among the m groups.

Here the term "alkylene group" refers to bivalent saturated hydrocarbon group and the term "arylene group" refers to bivalent aromatic hydrocarbon group.

In exemplary embodiments, the compound represented by Formula 1 above may include at least one of compounds represented by Formulas 2 to 4 below.

[Formula 2]

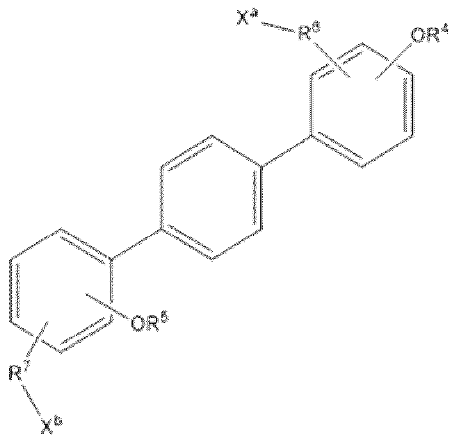

In Formula 2, $R^4$ and $R^5$ are each independently an organic group having 1 to 10 carbon atoms, $R^6$ and $R^7$ are each independently an organic group having 1 to 20 carbon atoms, which includes at least one of an alkylene group or an arylene group, and $X^a$ and $X^b$ are each independently halogen.

Here the term "the alkylene group" refers to bivalent saturated hydrocarbon group and the term "arylene group" refers to bivalent aromatic hydrocarbon group.

[Formula 3]

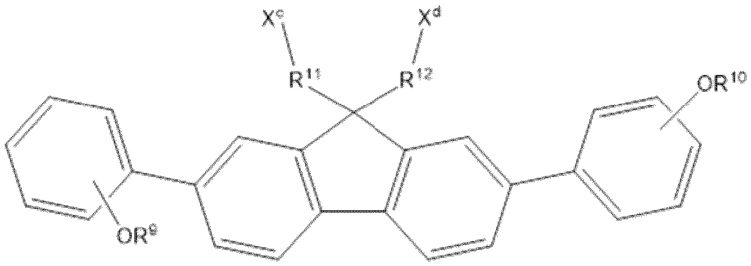

In Formula 3, $R^9$ and $R^{10}$ are each independently an organic group having 1 to 10 carbon atoms, $R^{11}$ and $R^{12}$ are each independently an organic group having 1 to 20 carbon atoms, which includes at least one of an alkylene group and/or an arylene group, and $X^c$ and $X^d$ are each independently halogen.

Here the term "the alkylene group" refers to bivalent saturated hydrocarbon group and the term "arylene group" refers to bivalent aromatic hydrocarbon group.

[Formula 4]

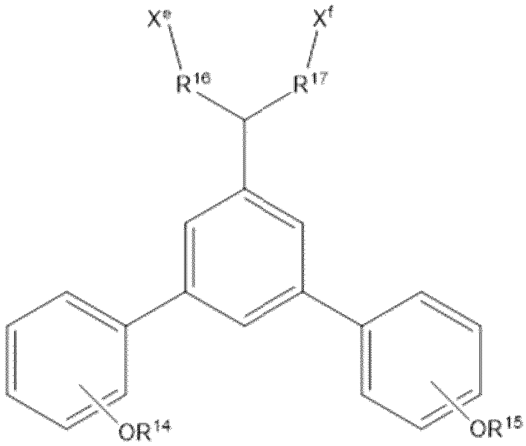

In Formula 4, $R^{14}$ and $R^{15}$ are each independently an organic group having 1 to 10 carbon atoms, $R^{16}$ and $R^{17}$ are each independently an organic group having 1 to 20 carbon atoms, which includes at least one of an alkylene group and/or an arylene group, and $X^e$ and $X^f$ are each independently halogen.

Here the term "the alkylene group" refers to bivalent saturated hydrocarbon group and the term "arylene group" refers to bivalent aromatic hydrocarbon group.

In exemplary embodiments, the compounds represented by Formulas 2 to 4 above may include compounds represented by Formulas 2-1 to 4-1 below, respectively.

[Formula 2-1]

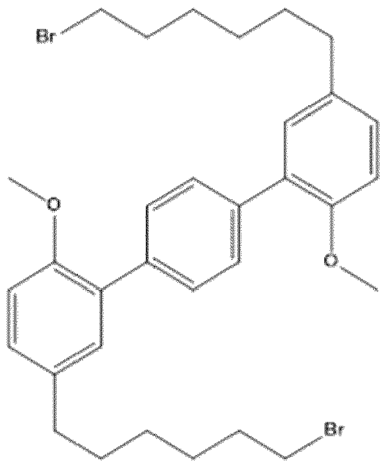

[Formula 3-1]

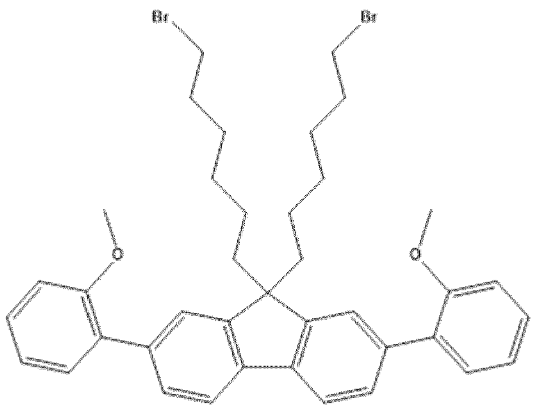

[Formula 4-1]

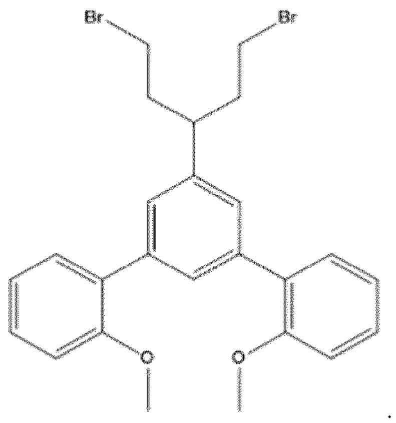

In exemplary embodiments, the compound may be a monomer for polymerization.

According to another aspect of the present invention, there is provided a precursor polymer including a repeating unit represented by Formula 1a below.

[Formula 1a]

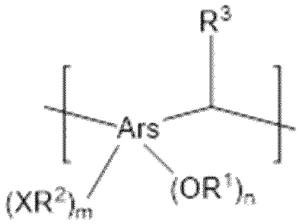

In Formula 1a, m and n are each independently an integer of 1 to 6, $R^1$ is each independently an organic group having 1 to 10 carbon atoms, $R^2$ is each independently an organic group having 1 to 20 carbon atoms, which includes at least one of an alkylene group and/or an arylene group, $R^3$ may be H or an organic group having 1 to 15 carbon atoms, X is each independently a halogen, and Ars is a polycyclic aromatic group having 10 to 50 carbon atoms.

Here the term "the alkylene group" refers to bivalent saturated hydrocarbon group and the term "arylene group" refers to bivalent aromatic hydrocarbon group.

In addition, according to another aspect of the present invention, there is provided an ion conductive polymer including a repeating unit represented by Formula 5 below.

[Formula 5]

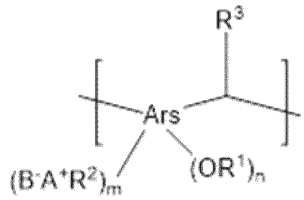

In Formula 5, $A^+$ is a quaternary ammonium, $B^-$ is an anion, m and n are each independently an integer of 1 to 6, $R^1$ is each independently an organic group having 1 to 10 carbon atoms, $R^2$ is each independently an organic group having 1 to 20 carbon atoms, which includes at least one of an alkylene group and/or an arylene group, $R^3$ may be H or an organic group having 1 to 15 carbon atoms, and Ars is a polycyclic- aromatic group having 10 to 50 carbon atoms.

Here the term "the alkylene group" refers to bivalent saturated hydrocarbon group and the term "arylene group" refers to bivalent aromatic hydrocarbon group.

In exemplary embodiments, the repeating unit represented by Formula 5 above may include at least one of repeating units represented by Formulas 6 to 8 below.

[Formula 6]

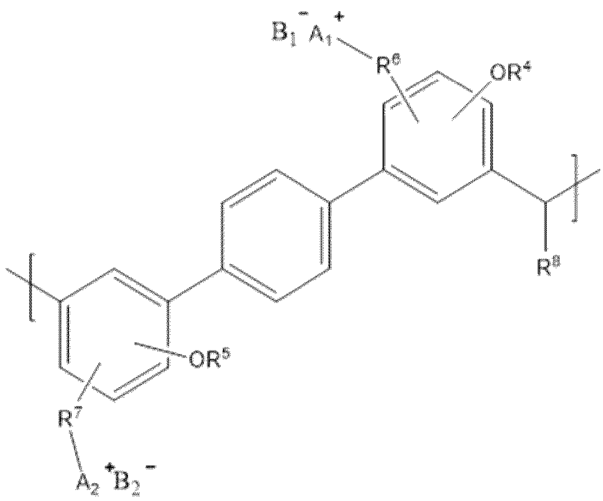

In Formula 6, $A_1^+$ and $A_2^+$ are each independently quaternary ammoniums, $B_1^-$ and $B_2^-$ are each independently anions, $R^4$ and $R^5$ are each independently an organic group having 1 to 10 carbon atoms, $R^6$ and $R^7$ are each independently an organic group having 1 to 20 carbon atoms, which includes at least one of an alkylene group and/or an arylene group, and $R^8$ may be H or an organic group having 1 to 15 carbon atoms.

Here the term "alkylene group" refers to bivalent saturated hydrocarbon group and the term "arylene group" refers to bivalent aromatic hydrocarbon group.

[Formula 7]

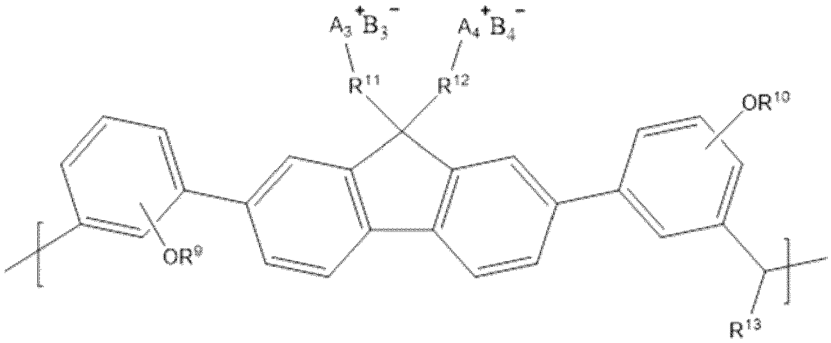

In Formula 7, $A_3^+$ and $A_4^+$ are each independently quaternary ammoniums, $B_3^-$ and $B_4^-$ are each independently anions, $R^9$ and $R^{10}$ are each independently an organic group having 1 to 10 carbon atoms, $R^{11}$ and $R^{12}$ are each independently an organic group having 1 to 20 carbon atoms, which includes at least one of an alkylene group and/or an arylene group, and $R^{13}$ may be H or an organic group having 1 to 15 carbon atoms.

Here the term "the alkylene group" refers to bivalent saturated hydrocarbon group and the term "arylene group" refers to bivalent aromatic hydrocarbon group.

[Formula 8]

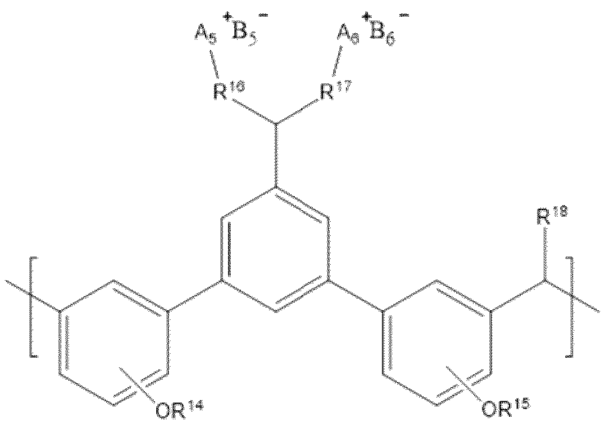

In Formula 8, $A_5^+$ and $A_6^+$ are each independently quaternary ammoniums, $B_5^-$ and $B_6^-$ are each independently anions, $R^{14}$ and $R^{15}$ are each independently an organic group having 1 to 10 carbon atoms, $R^{16}$ and $R^{17}$ are each independently an organic group having 1 to 20 carbon atoms, which includes at least one of an alkylene group and/or an arylene group, and $R^{18}$ may be H or an organic group having 1 to 15 carbon atoms.

Here the term "the alkylene group" refers to bivalent saturated hydrocarbon group and the term "arylene group" refers to bivalent aromatic hydrocarbon group.

In exemplary embodiments, the repeating units represented by Formulas 6 to 8 above may include repeating units represented by Formulas 6-1 to 8-1 below, respectively.

[Formula 6-1]

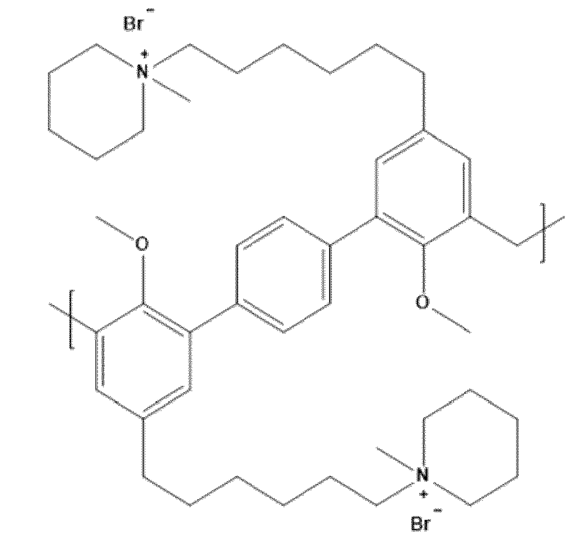

[Formula 7-1]

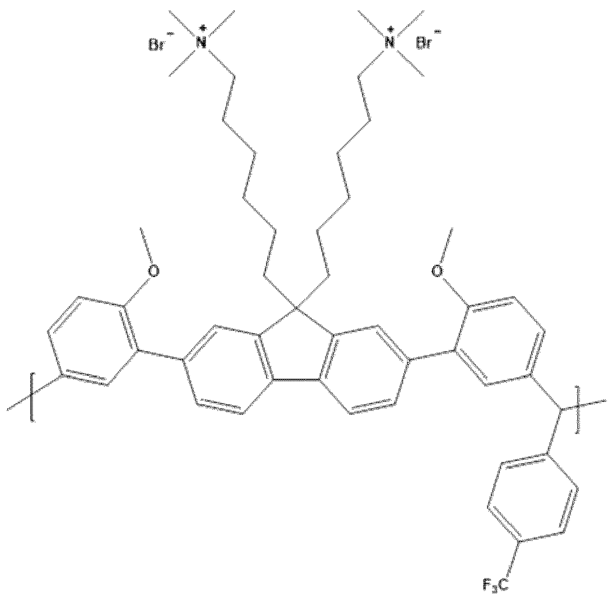

[Formula 8-1]

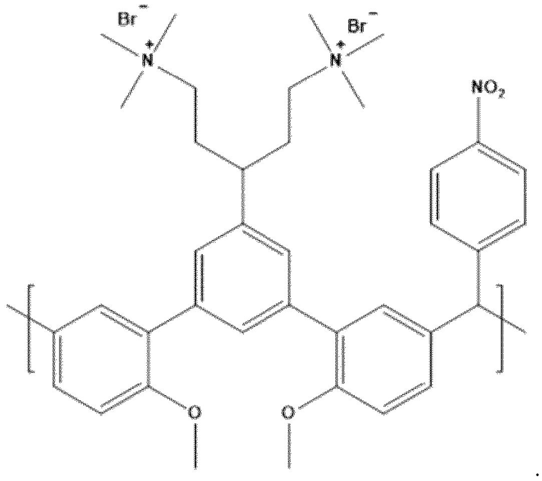

In exemplary embodiments, an $OH^-$ ionic conductivity of the ion conductive polymer at 25 °C may be 40 mS/cm to 80 mS/cm.

Further, according to another aspect of the present invention, there is provided a separator including the above-described ion conductive polymer.

Furthermore, according to another aspect of the present invention, there is provided a device including a cathode; an anode disposed to face the cathode; and the above-described separator.

In exemplary embodiments, the device may include a water electrolysis device, a $CO_2$ electrolysis device, a fuel cell, an electrolytic cell, and a vanadium flow battery.

The ion conductive polymer manufactured through the compound according to the exemplary embodiments of the present disclosure may have excellent ionic conductivity.

The separator manufactured through the compound may have excellent ionic conductivity.

The ion conductive polymer and separator manufactured through the compound of the present disclosure may be widely applied to green technology fields such as a water electrolysis device, a $CO_2$ electrolysis device and the other devices including the separator.

According to the embodiments of the present disclosure, a compound including an organic group in which at least one hydrogen is substituted with a halogen, an ether group and/or a polycyclic-aromatic group is provided.

As the compound includes the organic group in which at least one hydrogen is substituted with a halogen, at least one quaternary ammonium functional group may be introduced into an ion conductive polymer manufactured from the compound. Accordingly, electrochemical properties of the ion conductive polymer may be improved.

As the compound includes the ether group, the compound may be polymerized under mild acidic conditions.

As the compound includes the polycyclic aromatic group, it is possible to introduce the polycyclic aromatic group into a main chain of the ion conductive polymer manufactured from the compound. Accordingly, the mechanical strength and chemical resistance of the ion conductive polymer may be improved.

[DETAILED DESCRIPTION OF THE INVENTION]

Hereinafter, the present disclosure will be described in detail through embodiments with reference to the accompanying drawings. However, the embodiments are merely illustrative and the present disclosure is not limited to the specific embodiments described by way of example.

The term "organic group" as used herein may be a substituent which includes only carbon and hydrogen, or includes one or more atoms other than the carbon and hydrogen. For example, the atoms other than the carbon and hydrogen may be nitrogen (N), oxygen (O), phosphorus (P), sulfur (S), etc.

The term "polycyclic aromatic group" as used herein may mean a compound which includes two or more aromatic rings. For example, the polycyclic aromatic group may mean a compound in which compounds having two or more aromatic rings are consecutively linked, such as naphthalene, phenanthrene, etc., or a compound in which compounds having two or more aromatic rings are linked by a sigma (σ) bond, such as biphenyl, terphenyl, quaterphenyl, 2,7-bisphenylfluorene, etc.

The compound according to the exemplary embodiments of the present disclosure is represented by Formula 1 below.

[Formula 1]

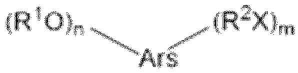

$R^1$ is each independently an organic group having 1 to 10 carbon atoms, which means that $R^1$ may be the same or different among the n groups. Further, $R^2$ is each independently an organic group having 1 to 20 carbon atoms, which means that $R^2$ may be the same or different among the m groups. In exemplary embodiments, m may be an integer of 1 to 6, 1 to 5, 1 to 3, 2 to 6, 2 to 5, or 2 to 4. For example, m may be 2.

If m is 7 or more, ammonium ($NH_4^+$) may be excessively introduced into the ion conductive polymer manufactured by polymerization of the compound, thereby causing a decrease in the mechanical properties of the polymer film.

In exemplary embodiments, n may be an integer of 1 to 6, 1 to 5, or 1 to 3. For example, n may be 2.

If n is 7 or more, ionic conductivity of the ion conductive polymer manufactured by polymerization of the compound may be reduced.

In exemplary embodiments, $R^1$ may each independently be an organic group having 1 to 10 carbon atoms, 1 to 8 carbon atoms, 1 to 6 carbon atoms, or 1 to 3 carbon atoms. For example, the $R^1$ may be a methyl group ($-CH_3$). Accordingly, the synthesis of the precursor polymer to be described below may be performed under relatively mild acidic conditions.

If the number of carbon atoms in $R^1$ is 11 or more, the ionic conductivity of the ion conductive polymer manufactured by polymerization of the compound may be reduced.

In exemplary embodiments, $R^2$ may each independently be an organic group having 1 to 20 carbon atoms, 2 to 20 carbon atoms, 3 to 20 carbon atoms, 5 to 20 carbon atoms, or 6 to 20 carbon atoms, which includes at least one of an alkylene group and/or an arylene group, wherein the "alkylene group" refers to saturated hydrocarbon. Accordingly, the quaternary ammonium functional group introduced into the ion conductive polymer manufactured by polymerization of the compound and the polycyclic aromatic group included in the main chain may not be directly linked, thereby improving stability, including the mechanical strength, and chemical resistance of the ion conductive polymer.

In exemplary embodiments, the X is a halogen atom. For example, X may be fluorine (F), chlorine (Cl), bromine (Br), or iodine (I), etc. Accordingly, a quaternary ammonium functional group may be introduced into the ion conductive polymer to be described below.

For example, X may each independently be chlorine (Cl), bromine (Br), or iodine (I). In one embodiment, X may each independently be bromine (Br), or iodine (I). In one embodiment, X may be iodine (I). The larger the particle size of the halogen atom, the easier it is to introduce a quaternary ammonium functional group into the ion conductive polymer manufactured by polymerization of the compound.

In exemplary embodiments, the Ars may be a polycyclic aromatic group having 10 to 50 carbon atoms, 11 to 45 carbon atoms, 12 to 40 carbon atoms, or 15 to 30 carbon atoms. Accordingly, the compound may introduce the polycyclic aromatic group into the main chain of the ion conductive polymer manufactured by polymerization.

If the number of carbon atoms in Ars exceeds 50, the solubility of the compound in a solvent may be greatly reduced, thereby resulting in low polymerization efficiency.

In exemplary embodiments, the compound represented by Formula 1 above may include at least one of compounds represented by Formulas 2 to 4 below.

[Formula 2]

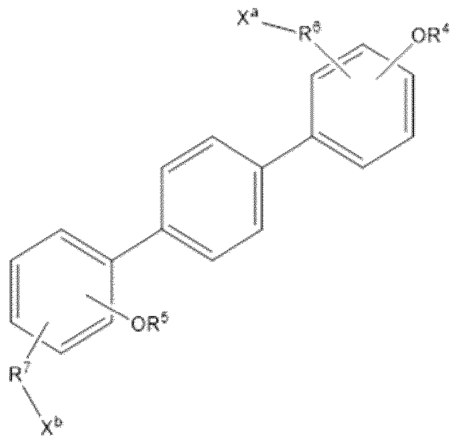

In exemplary embodiments, $R^4$ and $R^5$ may each independently be an organic group having 1 to 10 carbon atoms, 1 to 8 carbon atoms, 1 to 6 carbon atoms, or 1 to 3 carbon atoms. For example, the $R^4$ and $R^5$ may be a methyl group ($-CH_3$).

In exemplary embodiments, the $R^6$ and $R^7$ may each independently be an organic group having 1 to 20 carbon atoms, 2 to 20 carbon atoms, 3 to 20 carbon atoms, 5 to 20 carbon atoms, or 6 to 20 carbon atoms, which includes at least one of an alkylene group and/or an arylene group, wherein the alkylene group refers to bivalent saturated hydrocarbon group and the arylene group refers to bivalent aromatic hydrocarbon group.

In exemplary embodiments, the $X^a$ and $X^b$ are halogen atoms. For example, $X^a$ and $X^b$ may each independently be fluorine (F), chlorine (Cl), bromine (Br), or iodine (I), etc. For example, $X^a$ and $X^b$ may each independently be chlorine (Cl), bromine (Br), or iodine (I). In one embodiment, $X^a$ and $X^b$ may each independently be bromine (Br) and iodine (I). In one embodiment, both $X^a$ and $X^b$ may be iodine (I).

[Formula 3]

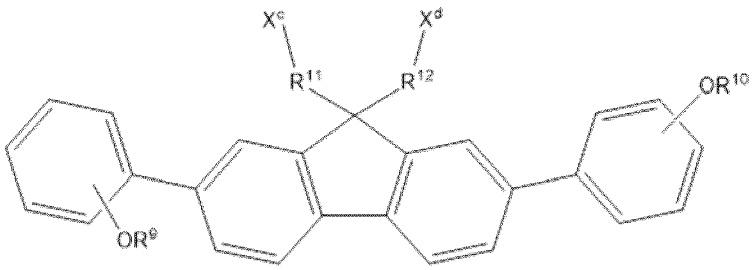

In exemplary embodiments, $R^9$ and $R^{10}$ may each independently be an organic group having 1 to 10 carbon atoms, 1 to 8 carbon atoms, 1 to 6 carbon atoms, or 1 to 3 carbon atoms. For example, the $R^9$ and $R^{10}$ may be a methyl group ($-CH_3$).

In exemplary embodiments, the $R^{11}$ and $R^{12}$ may each independently be an organic group having 1 to 20 carbon atoms, 2 to 20 carbon atoms, 3 to 20 carbon atoms, 5 to 20 carbon atoms, or 6 to 20 carbon atoms, which includes at least one of an alkylene group and/or an arylene group, wherein the alkylene group refers to bivalent saturated hydrocarbon group and the arylene group refers to bivalent aromatic hydrocarbon group.

In exemplary embodiments, the $X^c$ and $X^d$ are each independently halogen. For example, $X^c$ and $X^d$ may each independently be fluorine (F), chlorine (Cl), bromine (Br), or iodine (I), etc. For example, $X^c$ and $X^d$ may each independently be chlorine (Cl), bromine (Br), or iodine (I). In one embodiment, $X^c$ and $X^d$ may each independently be bromine (Br), or iodine (I). In one embodiment, both $X^c$ and $X^d$ may be iodine (I).

[Formula 4]

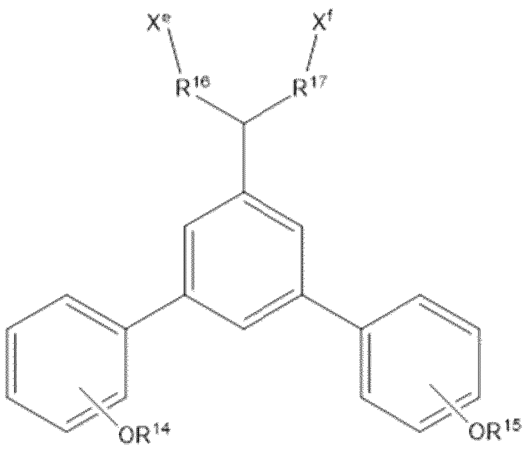

In exemplary embodiments, $R^{14}$ and $R^{15}$ may each independently be an organic group having 1 to 10 carbon atoms, 1 to 8 carbon atoms, 1 to 6 carbon atoms, or 1 to 3 carbon atoms. For example, the $R^{14}$ and $R^{15}$ may be a methyl group ($-CH_3$).

In exemplary embodiments, the $R^{16}$ and $R^{17}$ may each independently be an organic group having 1 to 20 carbon

atoms, 2 to 20 carbon atoms, 3 to 20 carbon atoms, 5 to 20 carbon atoms, or 6 to 20 carbon atoms, which includes at least one of an alkylene group and/or an arylene group, wherein the alkylene group refers to bivalent saturated hydrocarbon group and the arylene group refers to bivalent aromatic hydrocarbon group.

In exemplary embodiments, $X^e$ and $X^f$ are each independently halogen. For example, $X^e$ and $X^f$ may each independently be fluorine (F), chlorine (Cl), bromine (Br), or iodine (I), etc. For example, $X^e$ and $X^f$ may each independently be chlorine (Cl), bromine (Br), or iodine (I). In one embodiment, $X^e$ and $X^f$ may each independently be bromine (Br), or iodine (I). In one embodiment, both $X^e$ and $X^f$ may be iodine (I).

In exemplary embodiments, the compounds represented by Formulas 2 to 4 may be point symmetric or line symmetric with respect to a center point or center line (for example, middle phenyl among triphenyls in the case of Formula 2-1 or 4-1, or a center of cyclopentadiene or an imaginary center line passing through the center in the case of Formula 3-1) corresponding to a center of the compound. Accordingly, since both sides based on the center have the same reactivity, the synthesis and polymerization of the compound may be facilitated.

In exemplary embodiments, the compound represented by Formula 2 above may include a compound represented by Formula 2-1 below.

[Formula 2-1]

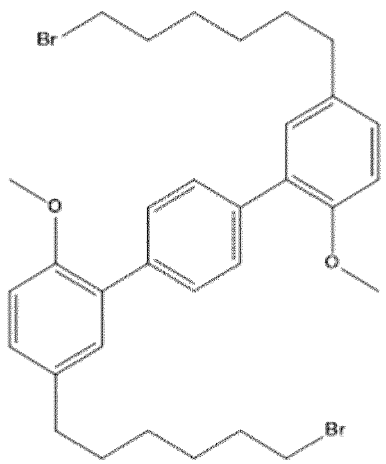

In exemplary embodiments, the compound represented by Formula 3 above may include a compound represented by Formula 3-1 below.

[Formula 3-1]

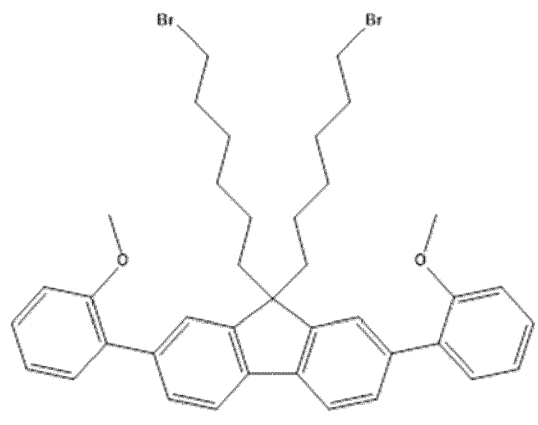

In exemplary embodiments, the compound represented by Formula 4 above may include a compound represented by Formula 4-1 below.

[Formula 4-1]

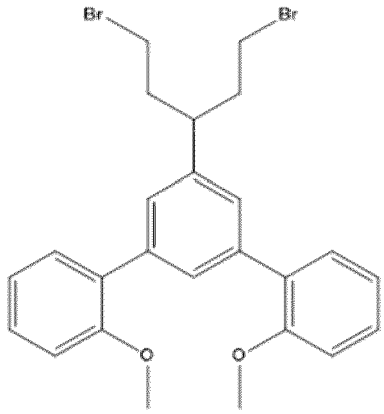

In exemplary embodiments, the compound represented by Formula 1 above may be a monomer for polymerization (hereinafter, may be abbreviated as a monomer). For example, the compound represented by Formula 1 above may be polymerized to become a precursor polymer of an ion conductive polymer (hereinafter, may be abbreviated as a precursor polymer).

In exemplary embodiments, the polymerization reaction may occur through a dehydration reaction in the presence of an acidic catalyst. For example, the polymerization reaction may be a Fridel-Crafts polycondensation of the compound represented by Formula 1 above with an aldehyde represented by $R^3$-CHO.

The precursor polymer according to exemplary embodiments of the present disclosure may be manufactured through polymerization of the monomer represented by Formula 1 above.

In exemplary embodiments, the precursor polymer may include a repeating unit represented by Formula 1a below.

[Formula 1a]

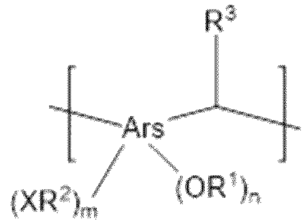

$R^1$ is each independently an organic group having 1 to 10 carbon atoms, which means that $R^1$ may be the same or different among the n groups. Further, $R^2$ is each independently an organic group having 1 to 20 carbon atoms, which means that $R^2$ may be the same or different among the m groups. In exemplary embodiments, m may be an integer of 1 to 6, 1 to 5, 1 to 3, 2 to 6, 2 to 5, or 2 to 4. For example, m may be 2. If m is 7 or more, ammonium ($NH_4^+$) may be excessively introduced into the ion conductive polymer manufactured from the precursor, thereby causing a decease in the mechanical properties of the film.

In exemplary embodiments, n may be an integer of 1 to 6, 1 to 5, or 1 to 3. For example, n may be 2.

If n is 7 or more, ionic conductivity of the ion conductive polymer manufactured from the precursor may be reduced.

In exemplary embodiments, $R^1$ may each independently be an organic group having 1 to 10 carbon atoms, 1 to 8 carbon atoms, 1 to 6 carbon atoms, or 1 to 3 carbon atoms. For example, the $R^1$ may be a methyl group ($-CH_3$).

If the number of carbon atoms in $R^1$ is 11 or more, the ionic conductivity of the ion conductive polymer manufactured from the precursor may be reduced.

In exemplary embodiments, $R^2$ may each independently be an organic group having 1 to 20 carbon atoms, 2 to 20 carbon atoms, 3 to 20 carbon atoms, 5 to 20 carbon atoms, or 6 to 20 carbon atoms, which includes at least one of an alkylene group and/or an arylene group, wherein the alkylene group refers to bivalent saturated hydrocarbon group and the arylene group refers to bivalent aromatic hydrocarbon group. Accordingly, the quaternary ammonium functional group introduced into the ion conductive polymer to be described below and the polycyclic aromatic group included in the main chain may not be directly linked, such that the stability, the mechanical strength and the chemical resistance of the ion

conductive polymer may be improved.

In exemplary embodiments, the $R^3$ may be H or an organic group having 1 to 15 carbon atoms, 1 to 10 carbon atoms, or 1 to 8 carbon atoms. For example, the $R^3$ may be H. Accordingly, the synthesis of the precursor polymer may be facilitated.

In exemplary embodiments, when the $R^3$ is an organic group other than H, the organic group may be further substituted with an electron withdrawing group (EWG). The electron withdrawing group may be, for example, a nitro group, a trifluoromethyl group, a cyano group, a fluoro group, an acyl group, an alkylsulfonyl group, etc. Accordingly, even when $R^3$ is an organic group other than H, the synthesis of the precursor polymer may be facilitated.

In exemplary embodiments, the X is a halogen atom. For example, X may be fluorine (F), chlorine (Cl), bromine (Br), or iodine (I), etc. Accordingly, a quaternary ammonium functional group may be introduced into the ion conductive polymer to be described below.

For example, X may each independently be chlorine (Cl), bromine (Br), or iodine (I). In one embodiment, X may each independently be bromine (Br) and iodine (I). In one embodiment, X may be iodine (I). The larger the particle size of the halogen atom, the easier it is to introduce a quaternary ammonium functional group into the ion conductive polymer to be described below.

In exemplary embodiments, the Ars may be a polycyclic aromatic group having 10 to 50 carbon atoms, 11 to 45 carbon atoms, 12 to 40 carbon atoms, or 15 to 30 carbon atoms. Accordingly, the multi-aromatic rings may be positioned in the main chain of the ion conductive polymer to be described below.

In exemplary embodiments, a repeating unit represented by Formula 1a above may include at least one of repeating units represented by Formulas 2a to 4a below.

[Formula 2a]

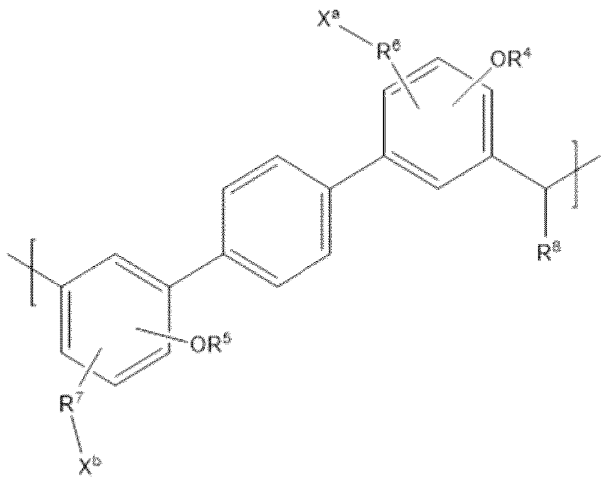

In exemplary embodiments, $R^4$ and $R^5$ may each independently be an organic group having 1 to 10 carbon atoms, 1 to 8 carbon atoms, 1 to 6 carbon atoms, or 1 to 3 carbon atoms. For example, the $R^4$ and $R^5$ may be a methyl group ($-CH_3$).

In exemplary embodiments, the $R^6$ and $R^7$ may each independently be an organic group having 1 to 20 carbon atoms, 2 to 20 carbon atoms, 3 to 20 carbon atoms, 5 to 20 carbon atoms, or 6 to 20 carbon atoms, which includes at least one of an alkylene group and/or an arylene group, wherein the alkylene group refers to bivalent saturated hydrocarbon group and the arylene group refers to bivalent aromatic hydrocarbon group.

In exemplary embodiments, the $R^8$ may be H or an organic group having 1 to 15 carbon atoms, 1 to 10 carbon atoms, or 1 to 8 carbon atoms.

In exemplary embodiments, the $X^a$ and $X^b$ are halogen atoms. For example, $X^a$ and $X^b$ may each independently be fluorine (F), chlorine (Cl), bromine (Br), or iodine (I), etc. For example, $X^a$ and $X^b$ may each independently be chlorine (Cl), bromine (Br), or iodine (I). In one embodiment, $X^a$ and $X^b$ may each independently be bromine (Br), or iodine (I). In one embodiment, both $X^a$ and $X^b$ may be iodine (I).

[Formula 3a]

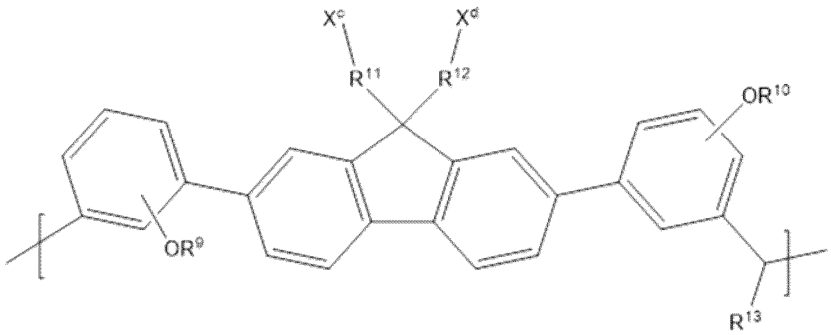

In exemplary embodiments, $R^9$ and $R^{10}$ may each independently be an organic group having 1 to 10 carbon atoms, 1 to 8 carbon atoms, 1 to 6 carbon atoms, or 1 to 3 carbon atoms. For example, the $R^9$ and $R^{10}$ may be a methyl group ($-CH_3$).

In exemplary embodiments, the $R^{11}$ and $R^{12}$ may each independently be an organic group having 1 to 20 carbon atoms, 2 to 20 carbon atoms, 3 to 20 carbon atoms, 5 to 20 carbon atoms, or 6 to 20 carbon atoms, which includes at least one of an alkylene group and/or an arylene group, wherein the alkylene group refers to bivalent saturated hydrocarbon group and the arylene group refers to bivalent aromatic hydrocarbon group.

In exemplary embodiments, the $R^{13}$ may be H or an organic group having 1 to 15 carbon atoms, 1 to 10 carbon atoms, or 1 to 8 carbon atoms.

In exemplary embodiments, the $X^c$ and $X^d$ are halogen atoms. For example, $X^c$ and $X^d$ may each independently be fluorine (F), chlorine (Cl), bromine (Br), or iodine (I), etc. For example, $X^c$ and $X^d$ may each independently be chlorine (Cl), bromine (Br), or iodine (I). In one embodiment, $X^c$ and $X^d$ may each independently be bromine (Br), or iodine (I). In one embodiment, both $X^c$ and $X^d$ may be iodine (I).

[Formula 4a]

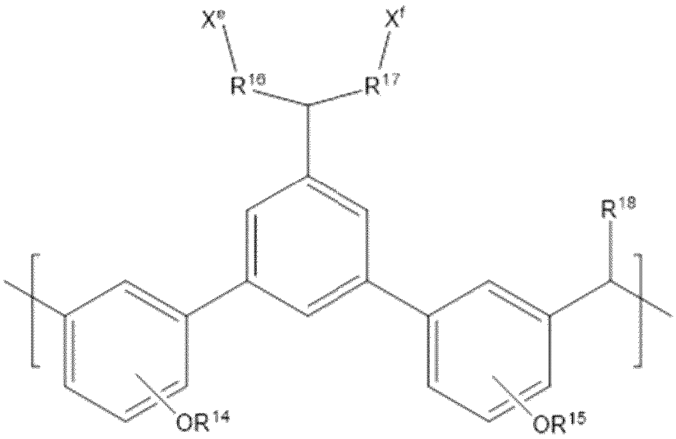

In exemplary embodiments, $R^{14}$ and $R^{15}$ may each independently be an organic group having 1 to 10 carbon atoms, 1 to 8 carbon atoms, 1 to 6 carbon atoms, or 1 to 3 carbon atoms. For example, the $R^{14}$ and $R^{15}$ may be a methyl group ($-CH_3$).

In exemplary embodiments, the $R^{16}$ and $R^{17}$ may each independently be an organic group having 1 to 20 carbon atoms, 2 to 20 carbon atoms, 3 to 20 carbon atoms, 5 to 20 carbon atoms, or 6 to 20 carbon atoms, which includes at least one of an alkylene group and/or an arylene group, wherein the alkylene group refers to bivalent saturated hydrocarbon group and the arylene group refers to bivalent aromatic hydrocarbon group.

In exemplary embodiments, the $R^{18}$ may be H or an organic group having 1 to 15 carbon atoms, 1 to 10 carbon atoms, or 1 to 8 carbon atoms.

In exemplary embodiments, the $X^e$ and $X^f$ are each independently halogen atoms. For example, $X^e$ and $X^f$ may each independently be fluorine (F), chlorine (Cl), bromine (Br), or iodine (I), etc. For example, $X^e$ and $X^f$ may each independently be chlorine (Cl), bromine (Br), or iodine (I). In one embodiment, $X^e$ and $X^f$ may each independently be bromine (Br), or iodine (I). In one embodiment, both $X^e$ and $X^f$ may be iodine (I).

In exemplary embodiments, the repeating unit represented by Formula 2a above may include a repeating unit represented by Formula 2a-1 below.

[Formula 2a-1]

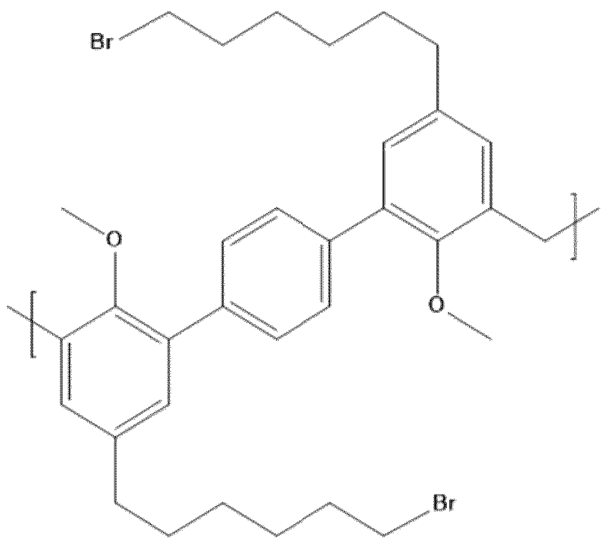

In exemplary embodiments, the repeating unit represented by Formula 3a above may include a repeating unit represented by Formula 3a-1 below.

[Formula 3a-1]

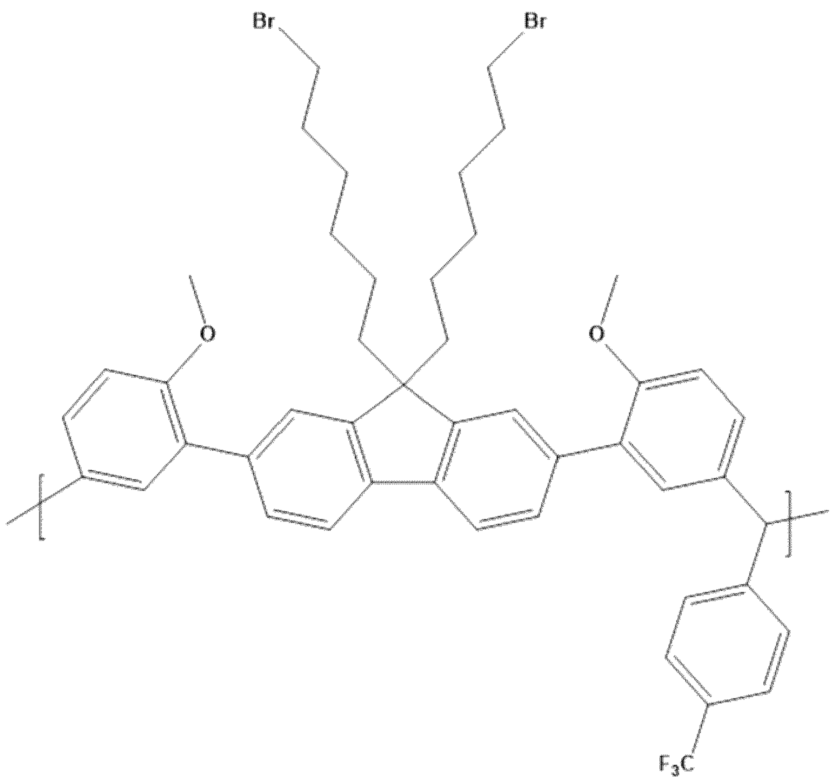

In exemplary embodiments, the repeating unit represented by Formula 4a above may include a repeating unit represented by Formula 4a-1 below.

[Formula 4a-1]

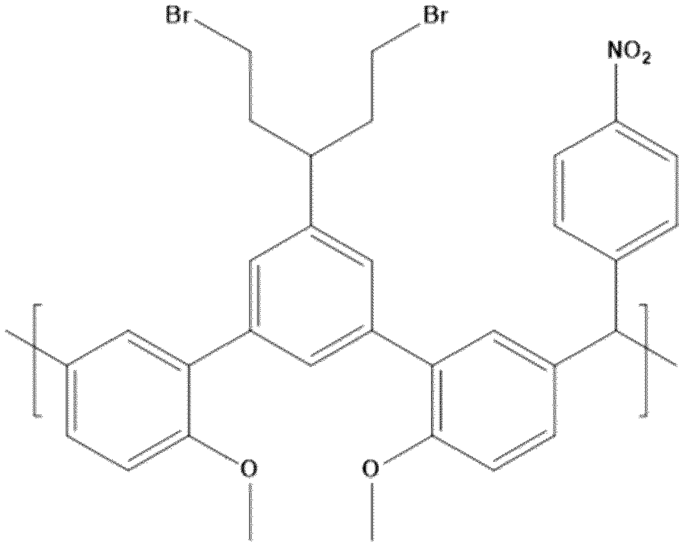

The ion conductive polymer according to exemplary embodiments of the present disclosure may be manufactured from a precursor polymer including the repeating unit represented by Formula 1a above.

For example, the ion conductive polymer may be manufactured through a bimolecular nucleophilic substitution reaction ($S_N2$ reaction) between the precursor polymer including the repeating unit represented by Formula 1a above and a tertiary amine compound. As a result of the reaction, the quaternary ammonium group may be formed.

As the tertiary amine compound, N,N-dimethylformamide, trimethylamine, triethylamine, tripropylamine, tributylamine, imidazole, N-methylpiperidine, quinuclidine, or a similar tertiary amine compound may be used.

In exemplary embodiments, the ion conductive polymer includes a repeating unit represented by Formula 5 below.

[Formula 5]

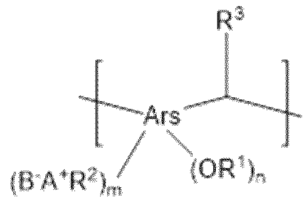

In exemplary embodiments, $A^+$ may be a quaternary ammonium (quaternary ammonium group). In exemplary embodiments, $A^+$ may be a quaternary ammonium which results from reacting the precursor polymer with the aforementioned tertiary amine compound. In exemplary embodiments, $A^+$ may be a quaternary ammonium including at least one methyl group.

In the present disclosure, the ammonium may also include ammonium in which two different substituents among substituents bonded to a nitrogen atom are linked to form a ring structure. For example, $A^+$ may be trimethylammonium, triethylammonium, tripropylammonium, tributylammonium, imidazolium, or N-methylpiperidinium, quinuclidinium.

In exemplary embodiments, $B^-$ may be an anion. In exemplary embodiments, $B^-$ may be a monovalent or divalent anion. For example, when $B^-$ is a divalent anion, two $A^+$ and $A^+$ located on different chains may bind together to one $B^-$ to form a cross-linked structure.

In exemplary embodiments, $B^-$ may be a halogen anion or a polyatomic anion. For example, $B^-$ may be a chloride anion ($Cl^-$), a bromine anion ($Br^-$), an iodine anion ($I^-$), a hydroxide ion ($OH^-$), a sulfate anion ($SO_4^{2-}$), a carbonate anion ($CO_3^{2-}$), a bicarbonate anion ($HCO_3^-$), or a carboxylic acid anion ($RCO_2^-$).

In exemplary embodiments, m may be an integer of 1 to 6, 1 to 5, 1 to 3, 2 to 6, 2 to 5, or 2 to 4. For example, m may be 2.

If m is 7 or more, mechanical properties of a film made of the ion conductive polymer may be decreased.

In exemplary embodiments, n may be an integer of 1 to 6, 1 to 5, or 1 to 3. For example, n may be 2.

If n is 7 or more, the ionic conductivity of the ion conductive polymer may be reduced.

In exemplary embodiments, the $R^1$ may be an organic group having 1 to 10 carbon atoms, 1 to 8 carbon atoms, 1

to 6 carbon atoms, or 1 to 3 carbon atoms. For example, the $R^1$ may be a methyl group ($-CH_3$).

If the number of carbon atoms in $R^1$ is 11 or more, the ionic conductivity of the ion conductive polymer may be reduced.

In exemplary embodiments, in the ion conductive polymer including the repeating unit represented by the Formula 5 above, at least one of hydrogens of a polycyclic aromatic group may be substituted with an ether functional group. When the aromatic group of the ion conductive polymer and the ether functional group are bonded, the aromatic group is placed in an electron-rich state. Accordingly, the synthesis of the ion conductive polymer may be performed under relatively mild acidic conditions rather than super-strong acidic conditions such as triflic acid.

According to exemplary embodiments, the mild acid may have an acid dissociation constant (pKa) of -7 or more.

For example, the mild acid may be methanesulfonic acid, trifluoroacetic acid, nitric acid, sulfuric acid, or hydrochloric acid.

In exemplary embodiments, the $R^2$ may be an organic group having 1 to 20 carbon atoms, 2 to 20 carbon atoms, 3 to 20 carbon atoms, 5 to 20 carbon atoms, or 6 to 20 carbon atoms, which includes at least one of an alkylene group and/or an arylene group, wherein the alkylene group refers to bivalent saturated hydrocarbon group and the arylene group refers to bivalent aromatic hydrocarbon group.

In the repeating unit represented by Formula 5, if the number of carbon atoms between the quaternary ammonium ion $A^+$ and the polycyclic aromatic group Ars is too small, the ammonium functional group may be decomposed. When the decomposition of the ammonium functional group occurs, the mechanical strength and ionic conductivity of the ion conductive polymer may be decreased.

In the present disclosure, since the quaternary ammonium ion is linked to the polycyclic aromatic group Ars through $R^2$, the electron withdrawing inductive effect and resonance effect, etc. by the aromatic group are reduced, such that the attack by $OH^-$ may be decreased. Accordingly, the decomposition of the ammonium functional group may be prevented.

In exemplary embodiments, the $R^2$ may have aromatic rings or aliphatic chains mixed together therein. Accordingly, the mechanical strength of the ion conductive polymer may be further improved.

In exemplary embodiments, the $R^3$ may be H or an organic group having 1 to 15, 1 to 10, or 1 to 8 carbon atoms. For example, the $R^3$ may be H.

In exemplary embodiments, when the $R^3$ is an organic group other than H, the organic group may be further substituted with an electron withdrawing group (EWG). The electron withdrawing group may be, for example, a nitro group, a trifluoromethyl group, a cyano group, a fluoro group, an acyl group, an alkylsulfonyl group, etc.

In exemplary embodiments, the Ars may be a polycyclic aromatic group having 10 to 50 carbon atoms, 11 to 45 carbon atoms, 12 to 40 carbon atoms, or 15 to 30 carbon atoms.

As the main chain of the ion conductive polymer includes the polycyclic aromatic group, the mechanical strength and chemical resistance of the ion conductive polymer may be improved.

In exemplary embodiments, the repeating unit represented by Formula 5 above may include at least one of repeating units represented by Formulas 6 to 8 below.

[Formula 6]

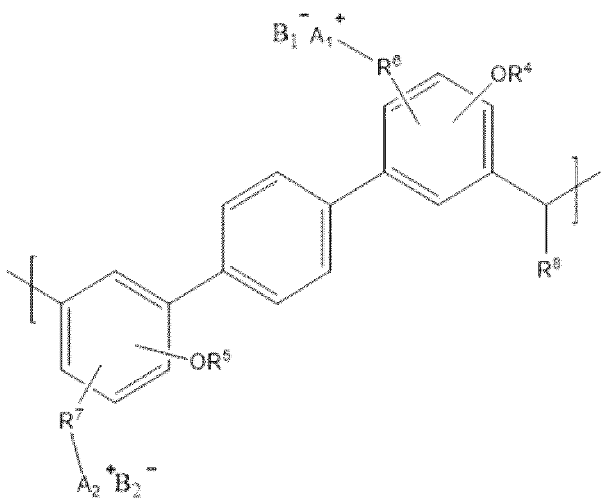

In exemplary embodiments, $A_1^+$ and $A_2^+$ may be quaternary ammoniums. In exemplary embodiments, $A_1^+$ and

$A_2^+$ may be quaternary ammoniums including at least one methyl group.

In exemplary embodiments, $B_1^-$ and $B_2^-$ may be anions. For example, $B_1^-$ and $B_2^-$ may each independently be a chloride anion ($Cl^-$), a bromine anion ($Br^-$), an iodine anion ($I^-$), a hydroxide ion ($OH^-$), a sulfate anion ($SO_4^{2-}$), a carbonate anion ($CO_3^{2-}$), a bicarbonate anion ($HCO_3^-$), or a carboxylic acid anion ($RCO_2^-$).

In exemplary embodiments, the $R^4$ and $R^5$ may each independently be an organic group having 1 to 10 carbon atoms, 1 to 8 carbon atoms, 1 to 6 carbon atoms, or 1 to 3 carbon atoms. For example, the $R^4$ and $R^5$ may be methyl groups ($-CH_3$).

In exemplary embodiments, the $R^6$ and $R^7$ may each independently be an organic group having 1 to 20 carbon atoms, 1 to 18 carbon atoms, 1 to 15 carbon atoms, or 1 to 10 carbon atoms, which includes at least one of an alkylene group and/or an arylene group, wherein the alkylene group refers to bivalent saturated hydrocarbon group and the arylene group refers to bivalent aromatic hydrocarbon group.

In exemplary embodiments, the $R^8$ may be H or an organic group having 1 to 15 carbon atoms, 1 to 10 carbon atoms, or 1 to 8 carbon atoms.

[Formula 7]

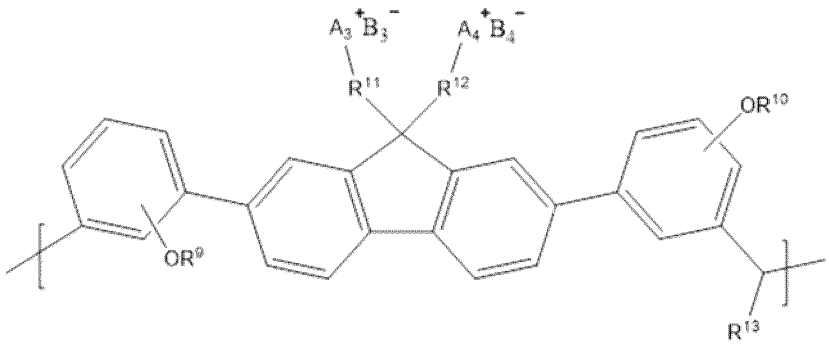

In exemplary embodiments, $A_3^+$ and $A_4^+$ may be quaternary ammoniums. In exemplary embodiments, $A_3^+$ and $A_4^+$ may be quaternary ammoniums including at least one methyl group.

In exemplary embodiments, $B_3^-$ and $B_4^-$ may be anions. For example, $B_3^-$ and $B_4^-$ may each independently be a chloride anion ($Cl^-$), a bromine anion ($Br^-$), an iodine anion ($I^-$), a hydroxide ion ($OH^-$), a sulfate anion ($SO_4^{2-}$), a carbonate anion ($CO_3^{2-}$), a bicarbonate anion ($HCO_3^-$), or a carboxylic acid anion ($RCO_2^-$).

In exemplary embodiments, $R^9$ and $R^{10}$ may each independently be an organic group having 1 to 10 carbon atoms, 1 to 8 carbon atoms, 1 to 6 carbon atoms, or 1 to 3 carbon atoms. For example, the $R^9$ and $R^{10}$ may be methyl groups ($-CH_3$).

In exemplary embodiments, the $R^{11}$ and $R^{12}$ may each independently be an organic group having 1 to 20 carbon atoms, 1 to 18 carbon atoms, 1 to 15 carbon atoms, or 1 to 10 carbon atoms, which includes at least one of an alkylene group and/or an arylene group, wherein the alkylene group refers to bivalent saturated hydrocarbon group and the arylene group refers to bivalent aromatic hydrocarbon group.

In exemplary embodiments, the $R^{13}$ may be H or an organic group having 1 to 15 carbon atoms, 1 to 10 carbon atoms, or 1 to 8 carbon atoms.

[Formula 8]

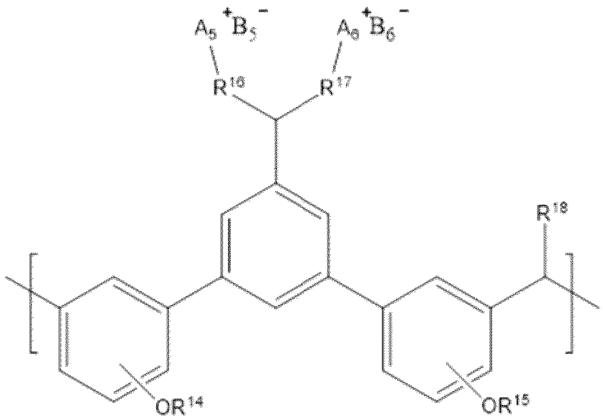

In exemplary embodiments, $A_5^+$ and $A_6^+$ may be quaternary ammoniums. In exemplary embodiments, $A_5^+$ and $A_6^+$ may be quaternary ammoniums including at least one methyl group.

In exemplary embodiments, $B_5^-$ and $B_6^-$ may be anions. For example, $B_5^-$ and $B_6^-$ may each independently be a chloride anion ($Cl^-$), a bromine anion ($Br^-$), an iodine anion ($I^-$), a hydroxide ion ($OH^-$), a sulfate anion ($SO_4^{2-}$), a carbonate anion ($CO_3^{2-}$), a bicarbonate anion ($HCO_3^-$), or a carboxylic acid anion ($RCO_2^-$).

In exemplary embodiments, $R^{14}$ and $R^{15}$ may each independently be an organic group having 1 to 10 carbon atoms, 1 to 8 carbon atoms, 1 to 6 carbon atoms, or 1 to 3 carbon atoms. For example, the $R^{14}$ and $R^{15}$ may be methyl groups ($-CH_3$).

In exemplary embodiments, the $R^{16}$ and $R^{17}$ may each independently be an organic group having 1 to 20 carbon atoms, 1 to 18 carbon atoms, 1 to 15 carbon atoms, or 1 to 10 carbon atoms, which includes at least one of an alkylene group and/or an arylene group, wherein the alkylene group refers to bivalent saturated hydrocarbon group and the arylene group refers to bivalent aromatic hydrocarbon group.

In exemplary embodiments, the $R^{18}$ may be H or an organic group having 1 to 15 carbon atoms, 1 to 10 carbon atoms, or 1 to 8 carbon atoms.

In exemplary embodiments, the repeating unit represented by Formula 6 above may include a repeating unit represented by Formula 6-1 below.

[Formula 6-1]

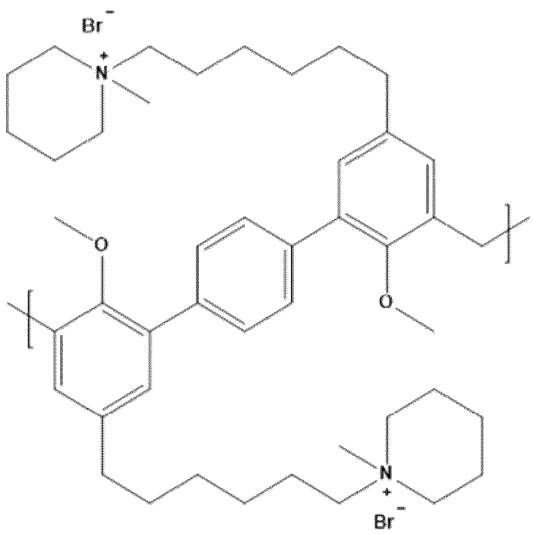

In exemplary embodiments, the repeating unit represented by Formula 7 above may include a repeating unit represented by Formula 7-1 below.

[Formula 7-1]

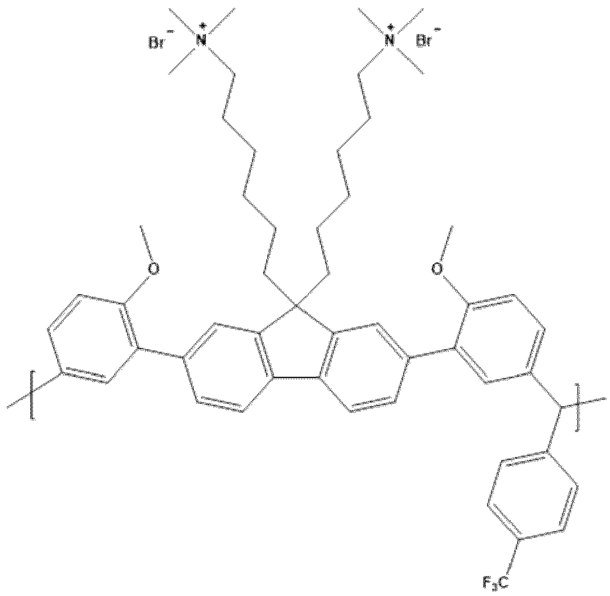

In exemplary embodiments, the repeating unit represented by Formula 8 above may include a repeating unit represented by Formula 8-1 below.

[Formula 8-1]

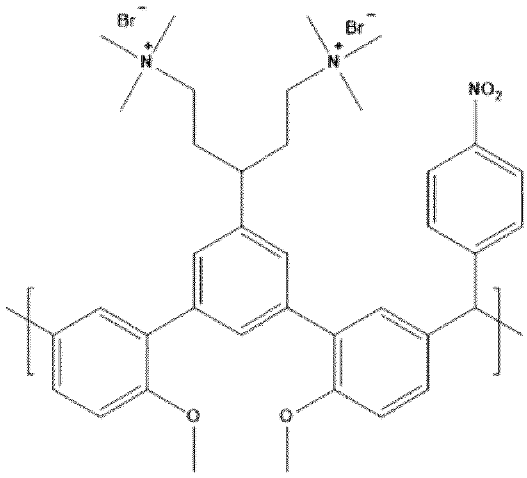

In exemplary embodiments, $OH^-$ ionic conductivity of the ion conductive polymer at 25 °C may be 40 mS/cm to 80 mS/cm. For example, the $OH^-$ ionic conductivity of the ion conductive polymer at 25 °C may be 43 mS/cm to 70 mS/cm, 45 mS/cm to 65 mS/cm, 46 mS/cm to 63 mS/cm, 47 mS/cm to 60 mS/cm, 53 mS/cm to 60 mS/cm, 53.5 mS/cm to 60 mS/cm, 54 mS/cm to 58 mS/cm.

Hereinafter, a method for manufacturing a monomer according to exemplary embodiments of the present disclosure is provided.

The method for manufacturing a monomer according to exemplary embodiments may include a mixed solution preparation step S10 and a coupling step S20.

The mixed solution preparation step may include a nitrogen ($N_2$) bubbling step. Accordingly, dissolved oxygen ($O_2$) inside the mixed solution may be removed.

The coupling step may be performed through an organic metal catalyst. As the organometallic catalyst, a palladium catalyst, or a nickel catalyst, etc. may be used. Accordingly, the monomer represented by Formula 1 above may be synthesized. The monomer represented by Formula 1 above may include an alkoxy group and a polycyclic aromatic group.

The alkoxy group may be - $(OR^1)_n$ in Formula 1 above. The n may be an integer of 1 to 6.

The polycyclic aromatic group may be bonded with $-(R^2A^+B^-)_m$ whose terminal is substituted with a halogen. The m may be an integer of 1 to 6.

The method for manufacturing an ion conductive polymer according to exemplary embodiments may include a monomer preparation step S30, a precursor synthesis step S40 and an ion conductive polymer synthesis step S50.

The monomer used in the monomer preparation step S30 may be obtained by purifying the monomer synthesized through the above-described monomer preparation method.

In the precursor synthesis step S40, the monomer may be reacted with an aldehyde under mild acidic conditions to obtain a precursor polymer.

When reacting the monomer with aldehyde under mild acidic conditions (primary polymerization), the polycyclic aromatic group of the monomer may be included in the main chain of the precursor polymer.

The primary polymerization may be carried out in the presence of a mild acid. As the mild acid, the above-described mild acids may be used. For example, an acid having a pKa of -7 or more may be used.

As described above, when at least one of the hydrogens of the benzene ring is substituted with an ether functional group, the benzene ring is placed in an electron-rich state. Accordingly, the synthesis of the ion conductive polymer may be performed under relatively mild acidic conditions rather than super-strong acidic conditions such as triflic acid.

The aldehyde may have $R^3$-CHO, and after the primary polymerization, it will have a structure in which $R^3$ is linked to the main chain of the precursor polymer.

In some embodiments, $R^3$ may be further substituted with an electron withdrawing group, and in this case, the precursor polymer may be more easily produced by increasing the reactivity of the aldehyde.

In the ion conductive polymer synthesis step S50, a quaternary ammonium group is introduced into the precursor polymer to synthesize an ion conductive polymer including the repeating unit represented by Formula 5 above.

When the quaternary ammonium group is introduced into a side chain of the multi-aromatic ring, the ion conductive polymer may exhibit improved ionic conductivity.

According to exemplary embodiments of the present disclosure, a separator including the ion conductive polymer is provided.

In exemplary embodiments, the separator may be manufactured by laminating ion conductive polymer films including the repeating unit represented by the Formula 5 above.

In exemplary embodiments, the separator may have a tensile strength of 10 MPa to 80 MPa, 15 MPa to 70 MPa, 20 MPa to 60 MPa, 25 MPa to 50 MPa, or 28 MPa to 45 MPa. The tensile strength may be measured using a universal testing machine (UTM) in an environment of 25 °C and a relative humidity of 50%.

According to embodiments of the present disclosure, a device including the separator is provided. The device may include: a cathode; an anode disposed to face the cathode; and the separator disposed between the cathode and the anode. The device according to exemplary embodiments may include a water electrolysis device, a $CO_2$ electrolysis device, a fuel cell, an electrolytic cell, a vanadium flow battery and other devices including a separator or similar devices.

Hereinafter, examples are proposed to facilitate understanding of the present disclosure, but these examples are only given for illustrating the present disclosure and are not intended to limit the appended claims. It will be apparent to those skilled in the art that various alterations and modifications are possible within the scope of the present disclosure, and such alterations and modifications are duly included in the appended claims.

## Preparative Example 1: Preparation of Monomer 1 (A-1)

### Example 1

20 mL of 1,2-dimethoxyethane, 20 mL of distilled water, 1,4-benzenediboronic acid (1.0 g, 6 mmol), 2-bromo-4-(6-bromohexyl)anisole (4.6 g, 13 mmol), and sodium carbonate ($Na_2CO_3$, 3.8 g, 36 mmol) were added to a 100 mL round bottom flask, followed by bubbling with nitrogen ($N_2$) for 30 minutes while stirring to prepare a mixed solution.

Tetrakis(triphenylphosphine)palladium(0)($Pd(PPh_3)_4$, 418 mg, 0.4 mmol) was added to the prepared mixed solution while maintaining a nitrogen ($N_2$) atmosphere, and refluxed for 24 hours by increasing the temperature to 80 °C. After refluxing, the mixture was cooled to room temperature, extracted twice with 20 mL of dichloromethane, and then moisture was removed with magnesium sulfate and concentrated under reduced pressure to obtain a solid.

The solid was purified by column chromatography to prepare 3.5 g of a compound of Example 1 represented by Formula 2-1 below as Monomer (A-1).

[Formula 2-1]

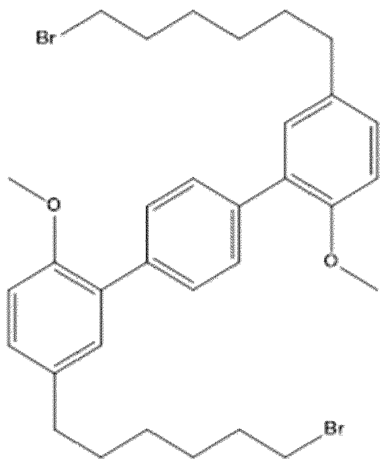

Results of $^1$H-nuclear magnetic resonance ($^1$H-NMR) spectroscopy analysis performed on the prepared A-1 are as follows.

$^1$H-NMR($CDCl_3$, ppm): 7.57(s, 4H), 7.19(d, 2H), 7.12(dd, 2H), 6.91(d, 2H), 3.81(s, 6H), 3.41(t, 4H), 2.61(t, 4H), 1.86(m, 4H), 1.64(m, 4H), 1.47(m, 4H), 1.38(m, 4H)

**Example 2: Preparation of Monomer 2 (A-2)**

15 mL of 1,2-dimethoxyethane, 15 mL of distilled water, 2,7-dibromo-9,9-bis(6-bromohexyl)fluorine (3.0 g, 5 mmol), 2-methoxyphenylboronic acid (1.8 g, 12 mmol), and sodium carbonate ($Na_2CO_3$, 1.2 g, 12 mmol) were added to a 100 mL round bottom flask, followed by bubbling with nitrogen ($N_2$) for 30 minutes while stirring to prepare a mixed solution.

Tetrakis(triphenylphosphine)palladium(0)(Pd($PPh_3$)$_4$, 267 mg, 0.2 mmol) was added to the prepared mixed solution while maintaining a nitrogen ($N_2$) atmosphere, and refluxed for 24 hours by increasing the temperature to 80 °C. After refluxing, the mixture was cooled to room temperature, extracted twice with 20 mL of dichloromethane, and then moisture was removed with magnesium sulfate and concentrated under reduced pressure to obtain a solid.

The solid was purified by column chromatography to prepare 3.0 g of a compound of Example 2 represented by Formula 3-1 below as Monomer (A-2).

[Formula 3-1]

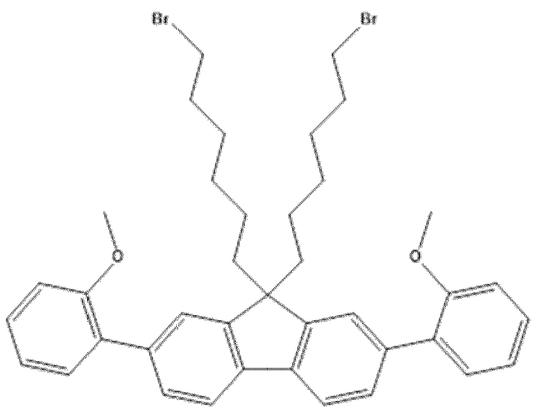

Results of $^1$H-nuclear magnetic resonance ($^1$H-NMR) spectroscopy analysis performed on the prepared A-2 are as follows.

$^1$H-NMR($CDCl_3$, ppm): 7.74(d, 2H), 7.52(m, 4H), 7.41(dd, 2H), 7.34(m, 2H), 7.07(t, 2H), 7.02(d, 2H), 3.84(s, 6H), 3.28(t, 4H), 1.99(m, 4H), 1.68(m, 4H), 1.23(m, 4H), 1.10(m, 4H), 0.84(m, 4H)

**Example 3: Preparation of Monomer 3 (A-3)**

15 mL of 1,2-dimethoxyethane, 15 mL of distilled water, 1,3-dibromo-5-(1,5-dibromopentane-3-yl)benzene (3.0 g, 6 mmol), 2-methoxyphenylboronic acid (2.5 g, 16 mmol), and sodium carbonate ($Na_2CO_3$, 1.7 g, 16 mmol) were added

to a 100 mL round bottom flask, followed by bubbling with nitrogen ($N_2$) for 30 minutes while stirring to prepare a mixed solution.

Tetrakis(triphenylphosphine)palladium(0)($Pd(PPh_3)_4$, 374 mg, 0.3 mmol) was added to the prepared mixed solution while maintaining a nitrogen ($N_2$) atmosphere, and refluxed for 24 hours by increasing the temperature to 80 °C. After refluxing, the mixture was cooled to room temperature, extracted twice with 20 mL of dichloromethane, and then moisture was removed with magnesium sulfate and concentrated under reduced pressure to obtain a solid.

The solid was purified by column chromatography to prepare 2.7 g of a compound of Example 3 represented by Formula 4-1 below as Monomer (A-3).

[Formula 4-1]

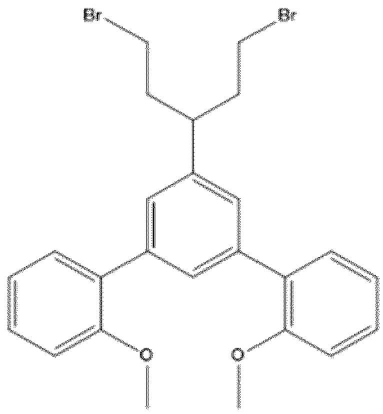

Results of $^1$H-nuclear magnetic resonance ($^1$H-NMR) spectroscopy analysis performed on the prepared A-3 are as follows.

$^1$H-NMR($CDCl_3$, ppm): 8.02(m, 4H), 7.84(s, 1H), 7.49(dd, 2H), 7.14(m, 4H), 3.79(s, 6H), 3.52(t, 4H), 2.58(m, 1H), 2.06(q, 4H)

**Preparative Example 2: Preparation of precursor polymer**

(1) Preparation of Precursor Polymer 1 (B-1) using the monomer of Example 1

20 mL of dichloromethane, 1.6 mL of methanesulfonic acid, paraformaldehyde (corresponding to 0.11 g, 3.6 mmol of formaldehyde), and the prepared A-1 (2 g, 3.2 mmol) were added to a 25 mL round bottom flask and stirred for 2 hours. After stirring, 200 mL of methanol was put therein to precipitate a solid. The precipitated solid was filtered and washed twice with 50 mL of methanol, then dried in an oven to prepare 1.8 g of Precursor Polymer 1 (B-1) represented by Formula 2a-1 below.

[Formula 2a-1]

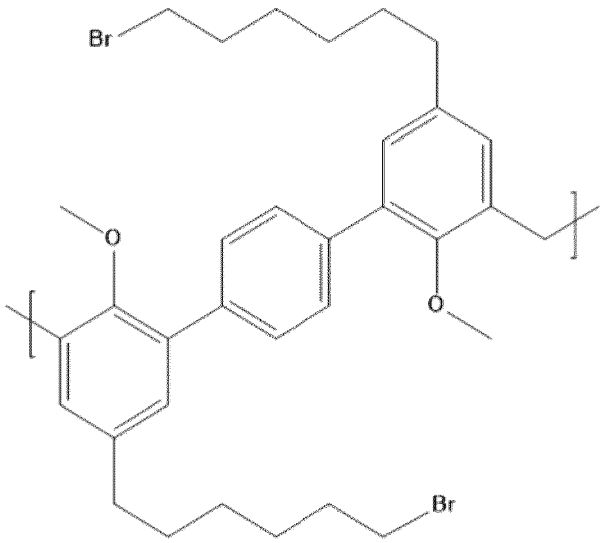

A weight average molar mass of the prepared B-1, measured by gel permeation chromatography (GPC) using polystyrene as a reference material, was 150,000 Da.

(2) Preparation of Precursor Polymer 2 (B-2) using the monomer of Example 2

12 mL of dichloromethane, 1 mL of methanesulfonic acid, 4-(trifluoromethyl)benzaldehyde (0.99 g, 5.7 mmol), and the prepared A-2 (2 g, 2.8 mmol) were added to a 25 mL round bottom flask and stirred for 8 hours. After stirring, 200 mL of methanol was put therein to precipitate a solid. The precipitated solid was filtered and washed twice with 50 mL of methanol, then dried in an oven to prepare 2.3 g of Precursor Polymer 2 (B-2) represented by Formula 3a-1 below.

[Formula 3a-1]

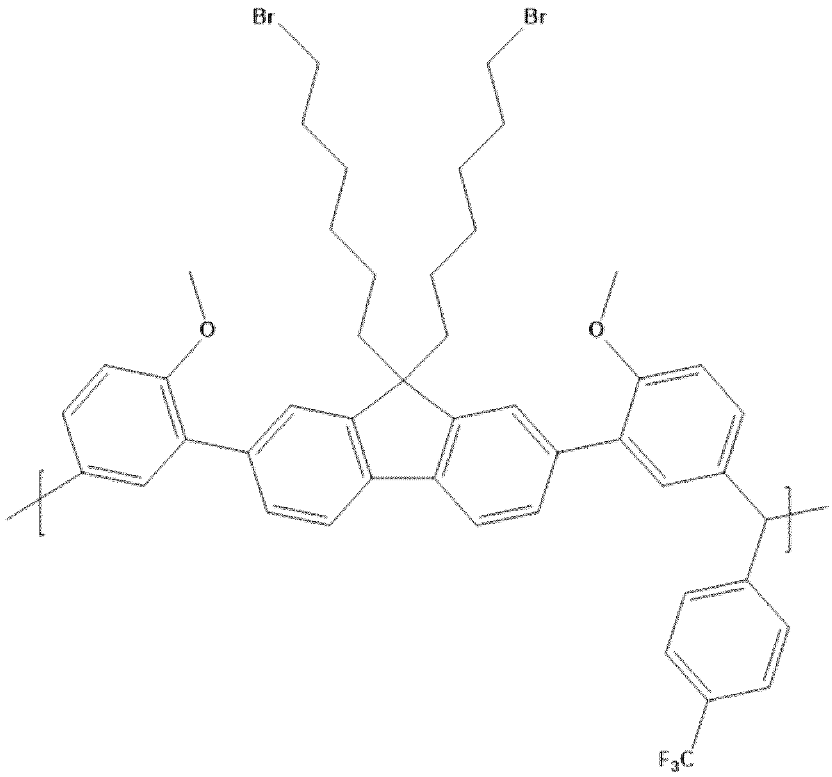

A weight average molar mass of the prepared B-2, measured by gel permeation chromatography (GPC) using

polystyrene as a reference material, was 210,000 Da.

(3) Preparation of Precursor Polymer 3 (B-3) using the monomer of Example 3

12 mL of dichloromethane, 3.7 mL of methanesulfonic acid, 4-nitrobenzaldehyde (0.7 g, 4.6 mmol), and the prepared A-3 (2 g, 3.9 mmol) were added to a 25 mL round bottom flask and stirred for 8 hours. After stirring, 200 mL of methanol was put therein to precipitate a solid. The precipitated solid was filtered and washed twice with 50 mL of methanol, then dried in an oven to prepare 2.4 g of Precursor Polymer 3 (B-3) represented by Formula 4a-1 below.

[Formula 4a-1]

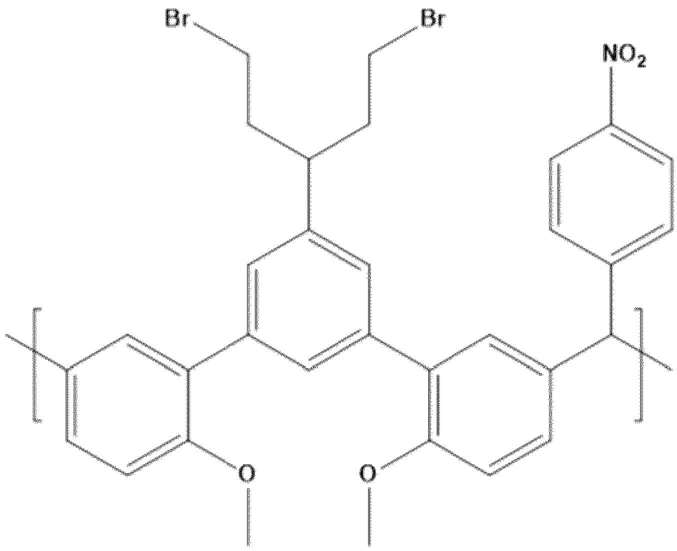

A weight average molar mass of the prepared B-3, measured by gel permeation chromatography (GPC) using polystyrene as a reference material, was 78,000 Da.

**Preparative Example 3: Manufacturing of ion conductive polymer film (separator)**

(1) Manufacturing of ion conductive polymer film (separator) (C-1) using the monomer of Example 1

2.2 mL of N,N-dimethylformamide and 0.3 g of the prepared B-1 were put into a 20 mL glass vial and stirred at room temperature (25 °C). After B-1 was completely dissolved, 0.12 g of N-methylpiperidine was added thereto and stirred at 80 °C for 24 hours. After stirring, the solution was poured into a petri dish and then dried in an oven at 80 °C for 24 hours to manufacture Separator (C-1) formed of a polymer including a repeating unit represented by Formula 6-1 below.

[Formula 6-1]

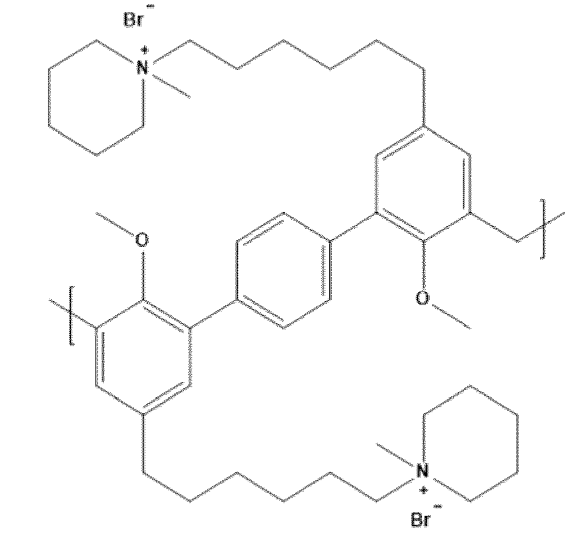

Results of $^1$H-nuclear magnetic resonance ($^1$H-NMR) spectroscopy analysis performed on the prepared C-1 are as follows.

$^1$H-NMR(DMSO-$d_6$, ppm): 7.72(br, 2H), 7.25(br, 4H), 7.23(br, 2H), 3.99(br, 2H), 3.79(br, 6H), 3.30(br, 6H), 3.22(br, 12H), 2.64(br, 4H), 1.71(br, 12H), 1.61(br, 8H), 1.29(br, 8H)

(2) Preparation of ion conductive polymer film (separator) (C-2) using the monomer of Example 2

2.6 mL of N,N-dimethylformamide and 0.3 g of the prepared B-2 were put into a 20 mL glass vial and stirred at room temperature (25 °C). After B-2 was completely dissolved, 0.46 g of a trimethylamine aqueous solution (28%) was added thereto and stirred at room temperature (25 °C) for 24 hours. After stirring, the solution was poured into a petri dish and then dried in an oven at 80 °C for 24 hours to manufacture Separator (C-2) formed of a polymer including a repeating unit represented by Formula 7-1 below.

[Formula 7-1]

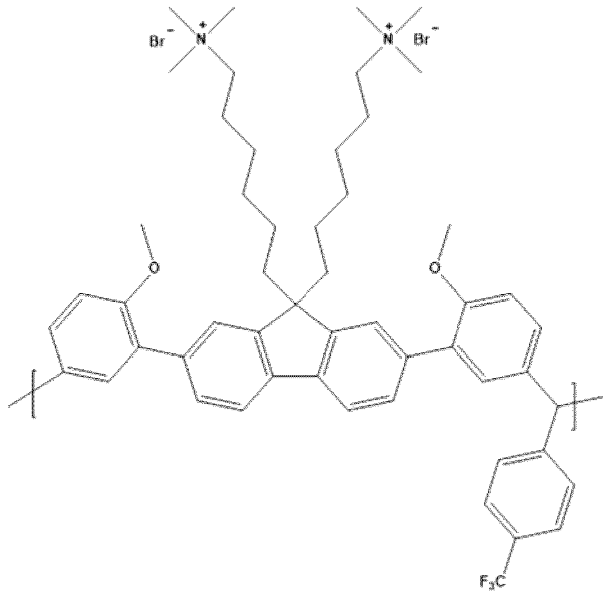

Results of $^1$H-nuclear magnetic resonance spectroscopy analysis performed on the prepared C-2 are as follows.

$^1$H-NMR(DMSO-$d_6$, ppm): 7.85(br, 2H), 7.75(br, 2H), 7.57(br, 2H), 7.51(br, 2H), 7.44(br, 2H), 7.28(br, 2H), 7.16(br, 4H), 5.91(br, 1H), 3.81(br, 6H), 3.57(br, 18H), 3.20(br, 4H), 1.97(br, 4H), 1.47(br, 4H), 1.06(br, 8H), 0.69(br, 4H)

(3) Preparation of ion conductive polymer film (separator) (C-3) using the monomer of Example 3

2.2 mL of N,N-dimethylformamide and 0.3 g of the prepared B-3 were put into a 20 mL glass vial and stirred at room temperature (25 °C). After B-3 was completely dissolved, 0.41 g of an aqueous trimethylamine solution (28%) was added thereto and stirred at room temperature (25 °C) for 24 hours. After stirring, the solution was poured into a petri dish and then dried in an oven at 80 °C for 24 hours to manufacture Separator (C-3) formed of a polymer including a repeating unit represented by Formula 8-1 below.

[Formula 8-1]

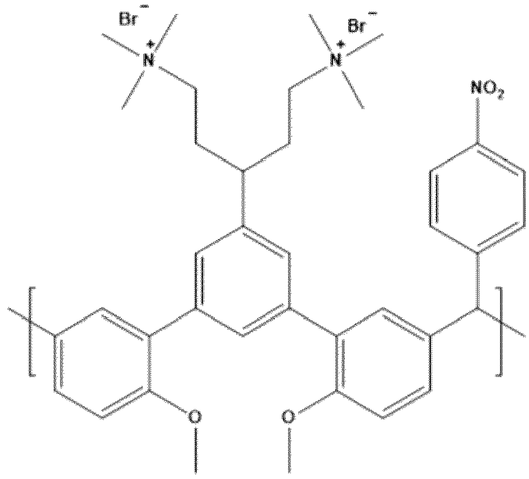

Results of $^{1}$H-nuclear magnetic resonance spectroscopy analysis performed on the prepared C-3 are as follows.
$^{1}$H-NMR(DMSO-$d_6$, ppm): 8.16(br, 2H), 8.04(br, 2H), 7.80(br, 3H), 7.44(br, 2H), 7.21(br, 2H), 7.14(br, 2H), 5.41(br, 1H), 3.79(br, 6H), 3.30(br, 18H), 3.22(br, 4H), 2.58(br, 1H), 2.00(br, 4H)

**Comparative Example 1**

Sustainion® X37-50 Grade RT product produced by Dioxide Materials was used.

**Comparative Example 2**

Sustainion® X37-50 Grade T product produced by Dioxide Materials was used.

**Experimental Example 1: Evaluation of ionic conductivity of ion conductive polymer film (separator)**

The separators manufactured using the monomers of Examples 1 to 3 and the membrane samples of Comparative Examples 1 to 2 were cut into a size of 1 cm × 3 cm and fixed between Pt electrodes of a Conductivity Clamp (BT-110, Scribner).
An ionic conductivity (σ) of an anion exchange membrane was evaluated in the form of $OH^-$ under conditions of room temperature (25 °C) and tertiary distilled water. A membrane resistance (R) was measured by means of a 4-point probe method using an impedance analyzer (VSP-3e, Biologics) in a frequency range of 0.1 kHz to 1 MHz. A thickness (T) of the membrane sample was measured using a micrometer.
The ionic conductivity (σ) was calculated using Equation 1 below, and results thereof are shown in Table 1 below.

[Equation 1]

$$\sigma = \frac{L}{R \times A} = \frac{L}{R \times W \times T}$$

In Equation 1, R is a membrane resistance (Ω), A is a cross-sectional area (cm$^2$) of the membrane sample, L is a distance (cm) between electrodes, W is a width (cm) of the membrane sample, and T is a thickness (cm) of the membrane

sample.

[TABLE 1]

| | $OH^-$ ionic conductivity (mS/cm) |
|---|---|
| Separator manufactured using monomer of Example 1 | 54.5 |
| Separator manufactured using monomer of Example 2 | 53.7 |
| Separator manufactured using monomer of Example 3 | 53.0 |
| Separator of Comparative Example 1 | 38.0 |
| Separator of Comparative Example 2 | 32.7 |

Referring to Table 1, it can be confirmed that the $OH^-$ ionic conductivities of the separators manufactured using the monomers of Examples 1 to 3 are superior to the $OH^-$ ionic conductivities of the separators of Comparative Examples 1 to 2. As demonstrated, when at least two quaternary ammoniums are bonded to a repeating unit (respectively the polycyclic aromatic group thereof), and respectively via hydrocarbon having some length of at least 2 carbon atoms (preferably more than 3 carbons) in embodiments based on Examples 1 to 3, the ionic conductivity thereof is superior to that in Comparative Example 1, in which only one quaternary ammonium is bonded to aromatic group via methylene.

**Experimental Example 2: Evaluation of tensile strength of ion conductive polymer film (separator)**

The separator samples manufactured using the monomers of Examples 1 to 3 were cut into a size of 1 cm × 6 cm, and tensile strengths were measured using a universal testing machine (Instron) at a crosshead speed of 10 mm/min in an environment of a temperature of 25 °C and a relative humidity of 50%, and results thereof are shown in Table 2 below.

[TABLE 2]

| | Tensile strength (MPa) |
|---|---|
| Separator manufactured using monomer of Example 1 | 28.1 |
| Separator manufactured using monomer of Example 2 | 42.3 |
| Separator manufactured using monomer of Example 3 | 35.9 |

Referring to Table 2, the tensile strengths of the separators manufactured using the monomers of Examples 1 to 3 were excellent at 28.1 MPa or more.

On the other hand, the strength of the separator of Comparative Example 1 was insufficient, thereby measurement of the tensile strength was impossible. This is attributed that the polycyclic aromatic group is introduced into a main chain in Examples 1 to 3 compared with Comparative Example 1, the polymer in which aromatic ring is located in the side chain.

The separator of Comparative Example 2 is a reinforced film comprising porous PTFE(Polytetrafluoroethylene) impregnated with a material of Comparative Example 1 to have a tensile strength corresponding to the separators of the Examples, but as described above, the $OH^-$ ionic conductivity was lower than that of the Examples.

## Claims

1. A compound represented by Formula 1 below:

[Formula 1]

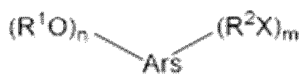

wherein in Formula 1, m and n are each independently an integer of 1 to 6, $R^1$ is each independently an organic group having 1 to 10 carbon atoms, $R^2$ is each independently an organic group having 1 to 20 carbon atoms, which includes

at least one of an alkylene group and/or an arylene group, X is each independently a halogen atom, and Ars is a polycyclic aromatic group having 10 to 50 carbon atoms.

**2.** The compound according to claim 1, wherein the compound represented by Formula 1 above comprises at least one of compounds represented by Formulas 2 to 4 below:

[Formula 2]

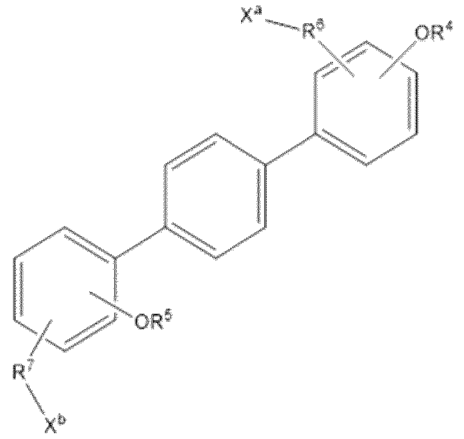

wherein in Formula 2, $R^4$ and $R^5$ are each independently an organic group having 1 to 10 carbon atoms, $R^6$ and $R^7$ are each independently an organic group having 1 to 20 carbon atoms, which includes at least one of an alkylene group and/or an arylene group, and $X^a$ and $X^b$ are each independently halogen atoms;

[Formula 3]

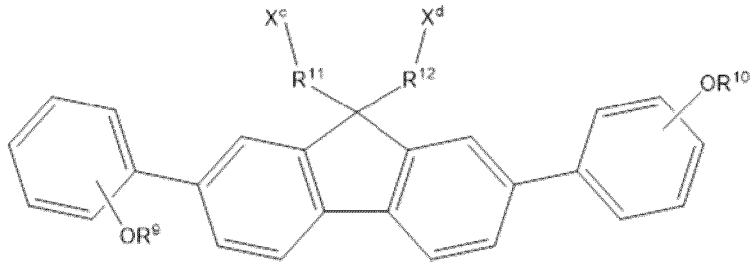

wherein in Formula 3, $R^9$ and $R^{10}$ are each independently an organic group having 1 to 10 carbon atoms, $R^{11}$ and $R^{12}$ are each independently an organic group having 1 to 20 carbon atoms, which includes at least one of an alkylene group and/or an arylene group, and $X^c$ and $X^d$ are each independently halogen atoms;

[Formula 4]

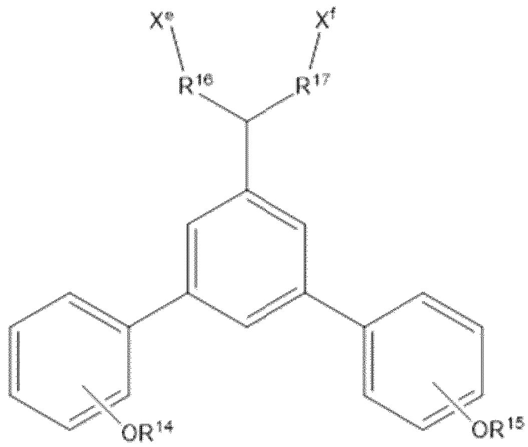

wherein in Formula 4, $R^{14}$ and $R^{15}$ are each independently an organic group having 1 to 10 carbon atoms, $R^{16}$ and $R^{17}$ are each independently an organic group having 1 to 20 carbon atoms, which includes at least one of an alkylene group and/or an arylene group, and $X^e$ and $X^f$ are each independently halogen atoms.

**3.** The compound according to claim 1 or 2, wherein the compounds represented by Formulas 2 to 4 above include compounds represented by Formulas 2-1 to 4-1 below, respectively:

[Formula 2-1]

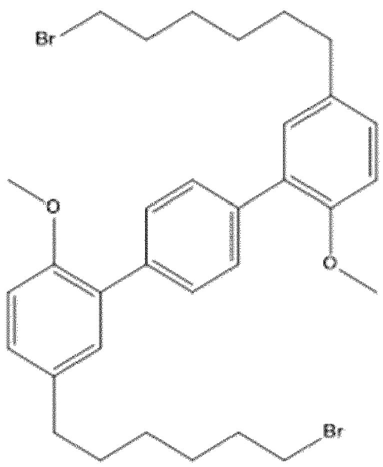

[Formula 3-1]

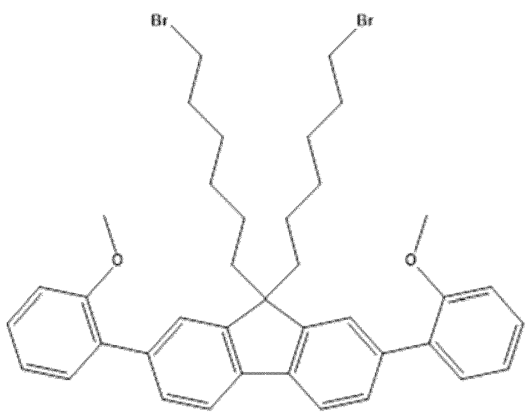

[Formula 4-1]

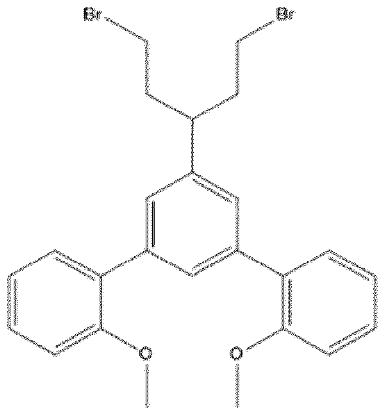

.

4. The compound according to any one of claims 1 to 3, wherein the compound is a monomer for polymerization.

5. A precursor polymer comprising a repeating unit represented by Formula 1a below:

[Formula 1a]

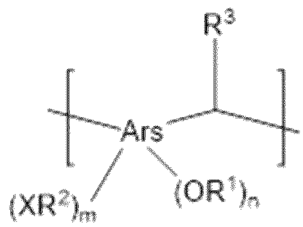

wherein in Formula 1a, m and n are each independently an integer of 1 to 6, $R^1$ is each independently an organic group having 1 to 10 carbon atoms, $R^2$ is each independently an organic group having 1 to 20 carbon atoms, which includes at least one of an alkylene group and/or an arylene group, $R^3$ optionally may be H or an organic group having 1 to 15 carbon atoms, X is each independently a halogen atom, and Ars is a polycyclic aromatic group having 10 to 50 carbon atoms.

6. An ion conductive polymer comprising a repeating unit represented by Formula 5 below:

[Formula 5]

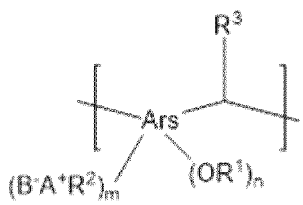

wherein in Formula 5, $A^+$ is a quaternary ammonium, $B^-$ is an anion, m and n are each independently an integer of 1 to 6, $R^1$ is each independently an organic group having 1 to 10 carbon atoms, $R^2$ is each independently an organic group having 1 to 20 carbon atoms, which includes at least one of an alkylene group and/or an arylene group, $R^3$ optionally may be H or an organic group having 1 to 15 carbon atoms, and Ars is a polycyclic aromatic group having 10 to 50 carbon atoms.

7. The ion conductive polymer according to claim 5 or 6, wherein the organic group of $R^3$ is present and is an electron withdrawing group (EWG), which is selected from the group consisting of a nitro group, a trifluoromethyl group, a

cyano group, a fluoro group, and an acyl group.

**8.** The ion conductive polymer according to claim 6 or 7, wherein
the repeating unit represented by Formula 5 above comprises at least one of repeating units represented by Formulas 6 to 8 below:

[Formula 6]

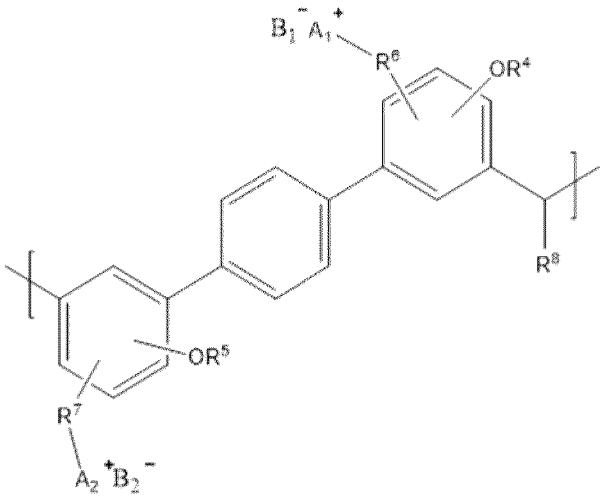

wherein in Formula 6, $A_1^+$ and $A_2^+$ are quaternary ammoniums, $B_1^-$ and $B_2^-$ are anions, $R^4$ and $R^5$ are each independently an organic group having 1 to 10 carbon atoms, $R^6$ and $R^7$ are each independently an organic group having 1 to 20 carbon atoms, which includes at least one of an alkylene group and/or an arylene group, and $R^8$ optionally may be H or an organic group having 1 to 15 carbon atoms;

[Formula 7]

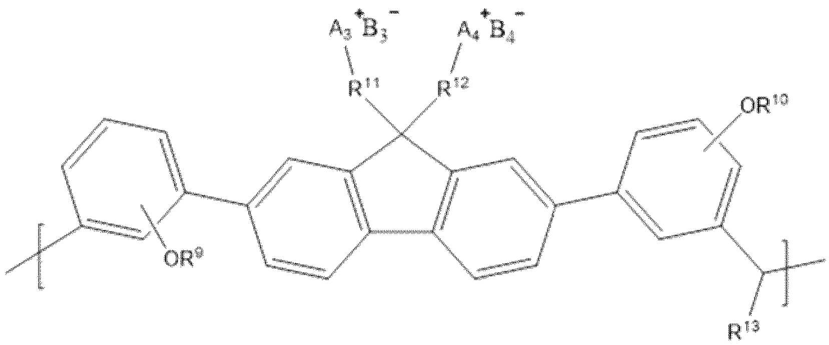

wherein in Formula 7, $A_3^+$ and $A_4^+$ are quaternary ammoniums, $B_3^-$ and $B_4^-$ are anions, $R^9$ and $R^{10}$ are each independently an organic group having 1 to 10 carbon atoms, $R^{11}$ and $R^{12}$ are each independently an organic group having 1 to 20 carbon atoms, which includes at least one of an alkylene group and/or an arylene group, and $R^{13}$ optionally may be H or an organic group having 1 to 15 carbon atoms;

[Formula 8]

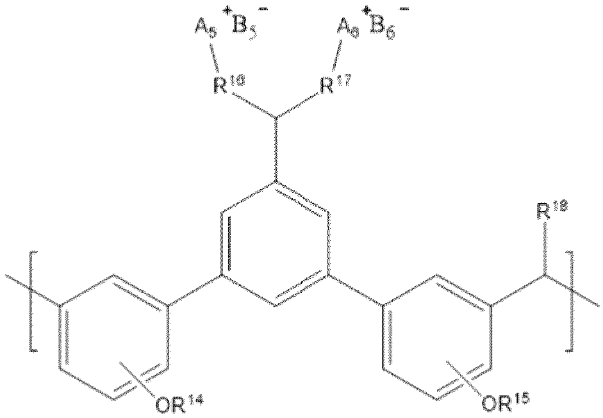

wherein in Formula 8, $A_5^+$ and $A_6^+$ are quaternary ammoniums, $B_5^-$ and $B_6^-$ are anions, $R^{14}$ and $R^{15}$ are each independently an organic group having 1 to 10 carbon atoms, $R^{16}$ and $R^{17}$ are each independently an organic group having 1 to 20 carbon atoms, which includes at least one of an alkylene group and/or an arylene group, and $R^{18}$ optionally may be H or an organic group having 1 to 15 carbon atoms.

**9.** The ion conductive polymer according to claim 8, wherein
the repeating units represented by Formulas 6 to 8 above include repeating units represented by Formulas 6-1 to 8-1 below, respectively:

[Formula 6-1]

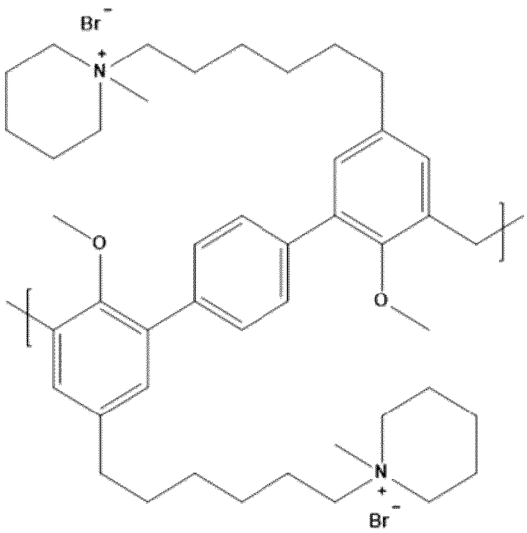

[Formula 7-1]

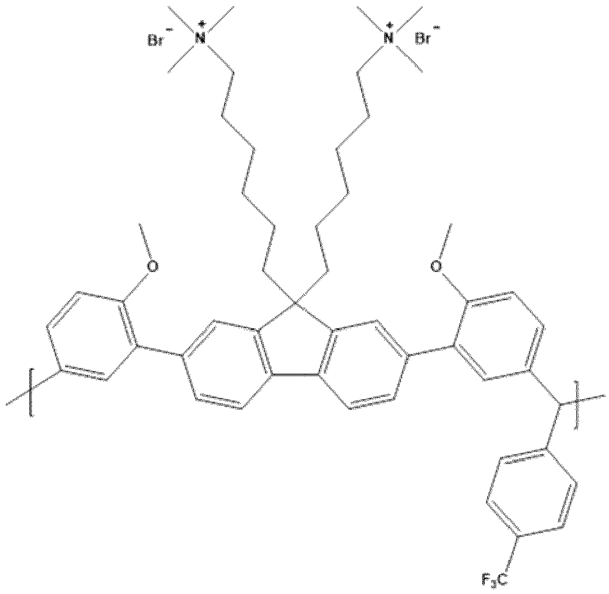

[Formula 8-1]

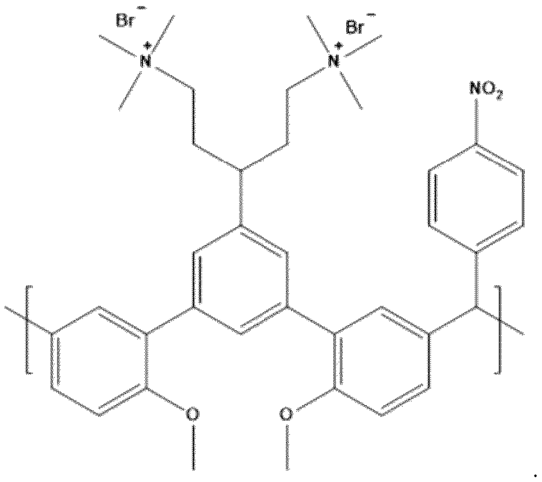

**10.** The ion conductive polymer according to any one of claims 6 to 9, wherein an $OH^-$ ionic conductivity of the ion conductive polymer at 25 °C is 40 mS/cm to 80 mS/cm.

**11.** The ion conductive polymer according to any one of claims 6 to 10, wherein the organic group of $R^2$ has 3 to 20 carbon atoms.

**12.** A separator comprising the ion conductive polymer of any one of claims 6 to 11.

**13.** The separator according to claim 12, wherein the separator is an ion exchange membrane.

**14.** A device comprising a cathode;

an anode disposed to face the cathode; and
the separator of claim 13.

**15.** The device according to claim 14, wherein the device comprises a water electrolysis device, a $CO_2$ electrolysis device, a fuel cell, an electrolytic cell, and a vanadium flow battery.

Europäisches Patentamt
European Patent Office
Office européen des brevets

# EUROPEAN SEARCH REPORT

Application Number
EP 25 19 1305

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | SAHA SAYANTANI ET AL: "New crosslinked sulfonated polytriazoles: Proton exchange properties and microbial fuel cell performance", EUROPEAN POLYMER JOURNAL, PERGAMON PRESS LTD OXFORD, GB, vol. 103, 22 April 2018 (2018-04-22), pages 322-334, XP085396965, ISSN: 0014-3057, DOI: 10.1016/J.EURPOLYMJ.2018.04.023 | 1,2,4 | INV. C07C43/225 C08F16/00 |
| Y | * page 2; compound TAZ * | 5-8, 10-15 | |
| A | | 3,9 | |
| X | JP 2013 161860 A (YAMAMOTO CHEMICALS INC) 19 August 2013 (2013-08-19) * page 25; compounds 4-1 * | 1 | |
| X | KR 2019 0132016 A (KOREA NATIONAL UNIV OF TRANSPORTATION INDUSTRY ACADEMIC COOPERATION FO) 27 November 2019 (2019-11-27) * page 28, paragraph [0165] - paragraph [0170] * | 1 | |

-/--

TECHNICAL FIELDS SEARCHED (IPC)

C07C
C08F

The present search report has been drawn up for all claims

1

| Place of search | Date of completion of the search | Examiner |
|---|---|---|
| The Hague | 23 December 2025 | Seitner, Irmgard |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 25 19 1305

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | YANG RUN ET AL: "Nonconjugated Self-Doped Polymer Zwitterions as Efficient Interlayers for High Performance Organic Solar Cells", CHEMISTRY OF MATERIALS, vol. 34, no. 16, 3 August 2022 (2022-08-03), pages 7293-7301, XP093348842, US ISSN: 0897-4756, DOI: 10.1021/acs.chemmater.2c01173 Retrieved from the Internet: URL:https://pubs.acs.org/doi/pdf/10.1021/acs.chemmater.2c01173> * page 2; compound 2 * ----- | 1 | |
| X | US 6 329 086 B1 (SHI JIANMIN [US] ET AL) 11 December 2001 (2001-12-11) * page 21 - page 23; compounds 4, 13, 16 and 21 * ----- | 1 | |
| X | BORIS BEHNISCH ET AL: "Luminescence of New 2,6- and 1,5-Naphthalene-Based PPV-TypeTrimers and Polymers", EUROPEAN JOURNAL OF ORGANIC CHEMISTRY, WILEY-VCH, DE, vol. 2000, no. 14, 7 September 2000 (2000-09-07), pages 2541-2549, XP072092214, ISSN: 1434-193X, DOI: 10.1002/1099-0690(200007)2000:14<2541::AID-EJOC2541>3.0.CO;2-J * page 2; compound 9 and 12 * ----- | 1 | TECHNICAL FIELDS SEARCHED (IPC) |

-/--

The present search report has been drawn up for all claims

1

| Place of search | Date of completion of the search | Examiner |
|---|---|---|
| The Hague | 23 December 2025 | Seitner, Irmgard |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 3

Europäisches Patentamt
European Patent Office
Office européen des brevets

# EUROPEAN SEARCH REPORT

Application Number
EP 25 19 1305

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | MISTRI ERSHAD ALI ET AL: "Structure-property correlation of semifluorinated 6-membered co-SPIs for proton exchange membrane", EUROPEAN POLYMER JOURNAL, PERGAMON PRESS LTD OXFORD, GB, vol. 73, 10 November 2015 (2015-11-10), pages 466-479, XP029305965, ISSN: 0014-3057, DOI: 10.1016/J.EURPOLYMJ.2015.11.006 * page 3 * ----- | 1 | |
| Y | REN RONG ET AL: "Facile Preparation of an Ether-Free Anion Exchange Membrane with Pendant Cyclic Quaternary Ammonium Groups", ACS APPLIED ENERGY MATERIALS, vol. 2, no. 7, 17 June 2019 (2019-06-17), pages 4576-4581, XP093059469, United States ISSN: 2574-0962, DOI: 10.1021/acsaem.9b00674 | 5-8, 10-15 | |
| A | * Scheme 1; page 2 * ----- | 3,9 | TECHNICAL FIELDS SEARCHED (IPC) |

The present search report has been drawn up for all claims

1

| Place of search | Date of completion of the search | Examiner |
|---|---|---|
| The Hague | 23 December 2025 | Seitner, Irmgard |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO.

EP 25 19 1305

This annex lists the patent family members relating to the patent documents cited in the above-mentioned European search report. The members are as contained in the European Patent Office EDP file on
The European Patent Office is in no way liable for these particulars which are merely given for the purpose of information.

23-12-2025

| Patent document cited in search report | Publication date | Patent family member(s) | Publication date |
|---|---|---|---|
| JP 2013161860 A | 19-08-2013 | NONE | |
| KR 20190132016 A | 27-11-2019 | NONE | |
| US 6329086 B1 | 11-12-2001 | DE 60100688 T2 | 22-07-2004 |
| | | EP 1164178 A1 | 19-12-2001 |
| | | JP 5662403 B2 | 28-01-2015 |
| | | JP 2002083683 A | 22-03-2002 |
| | | JP 2013051427 A | 14-03-2013 |
| | | KR 20010112635 A | 20-12-2001 |
| | | TW 530076 B | 01-05-2003 |
| | | US 6329086 B1 | 11-12-2001 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KR 20180100079